# EUROPEAN PATENT APPLICATION

(11) **EP 1 016 720 A1**
(43) Date of publication of application: **05.07.2000**
(21) Application number: 98941854.6
(22) Date of filing: 11.09.1998
(51) Int. Cl.: C12N 15/12, C12N 15/63, C12N 5/18, C07K 14/47, C12P 21/00

(54) **MAMMALIAN GENES PARTICIPATING IN CIRCADIAN PERIOD**

(30) Priority: 12.09.1997 JP 26784697
(71) Applicant: Sakaki, Yoshiyuki, Yokohama-shi, Kanagawa 236-0045 (JP)
(72) Inventor: SAKAKI, Yoshiyuki, Yokohama-shi, Kanagawa 236-0045 (JP); TEI, Hajime, Tokyo 113-0032 (JP)
(74) Representative: VOSSIUS & PARTNER
(86) International application number: JP9804125
(87) International publication number: WO9914324

(57) **Abstract**

A human gene and a mouse gene corresponding to *Drosophila* period gene which is known to be involved in the circadian period. The proteins and DNAs are applicable to the treatment of diseases relating to the circadian rhythm such as sleep phase delay syndorom, sleep phase progression syndrom, non-circadian sleep-wake syndrome, irregular sleep-wake disorder, and time difference syndrome (so-called jet lag), and to the labor and health management of irregular night time workers and the prevention of such disorders as night poriomania in dementia.

## Description

### Technical Field

The present invention relates to mammalian genes whose expression changes with a circadian period.

### Background Art

Many biochemical processes, physiological processes, and behavioral processes in various organisms ranging from microorganisms to vertebrates exhibit circadian rhythms (Edmunds, L. N. J., Cellular and Molecular Basis of Biological Clock, Springer-Verlag, New York, 1988). Several genes have been suggested to be involved in circadian rhythms.

For example, two mammalian circadian clock mutations have been confirmed thus far. They are Clock of the mouse (Vitaterna, M. H., et al., Science 264: 719-725, 1994) and tau of the hamster (Ralph, M. R. and Menaker, M., Science 241: 1225-1227, 1988). The Clock gene has recently been identified and is believed to encode a transcription factor in the circadian clock (Moor, R. Y. and Eichler, V. B., Brain Res. 42: 201-206: 1972; Stephan, F. K. and Zucker, I., Proc. Natl. Acad. Sci. USA 69: 1583-1586, 1972). On the other hand, the tau gene has not yet been cloned.

The period (per) gene has been isolated from *Drosophila* as a gene necessary for the expression of circadian rhythms for locomotive activities and eclosion behavior (Konopka, R. J. and Benzer, S., Proc. Natl. Acad. Sci. USA 68: 2112-2116, 1971). In the brain of the fly the oscillation of the levels of the per mRNA and of the PERIOD (dPER) protein are thought to determine the rhythms (Hardin, P. E., et al., Nature 343: 536-540, 1990; Zerr, D. M., et al., J. Neurosci. 10: 2749-2762, 1990). However, per homologues in other organisms than insects have not been identified.

### Disclosure of the Invention

An object of the present invention is to provide novel mammalian proteins and the genes thereof that are involved in the circadian period. More specifically, the object is to provide mammalian proteins and the genes thereof that are functionally equivalent to those of the *Drosophila* period (per) gene product.

To attain the above object, the present inventors focused on a region expected to play a functionally important role within the *Drosophila* gene known to be involved in the circadian rhythms, and performed a type of PCR, which had been developed on our own, using the primers designed based on the sequence of the region. As a result, we succeeded in isolating a human gene that corresponds to the above-mentioned *Drosophila* gene. We also succeeded in isolating a mouse gene that corresponds to the human gene by using the isolated human gene as a probe. Furthermore, we analyzed structures of the proteins encoded by the human and the mouse genes thus isolated and discovered that these proteins highly conserve the functional domains and the structural domains that have been identified in the *Drosophila* protein. In addition, analysis of the expression of the isolated mouse gene in the suprachiasmatic nucleus, which is the region responsible for functioning as a circadian pacemaker in the mammalian brain, revealed that the expression of the gene fluctuates with a circadian period.

Namely, the present invention relates to proteins and the genes thereof that are involved in the circadian periods of mammals, and more specifically to
(1) a protein derived from a mammal whose expression level in the suprachiasmatic nucleus (SCN) fluctuates with a circadian period,
(2) a protein of (1) wherein the mammal is a human,
(3) a protein of (1) wherein the mammal is a mouse,
(4) a protein involved in the formation of circadian rhythm in the suprachiasmatic nucleus (SCN) comprising the amino acid sequence described in SEQ ID NO: 1 or said sequence in which one or more amino acids are substituted, deleted, or added,
(5) a protein involved in the formation of circadian rhythm in the suprachiasmatic nucleus (SCN) comprising the amino acid sequence described in SEQ ID NO: 2 or said sequence in which one or more amino acids are substituted, deleted, or added,
(6) a protein involved in the formation of circadian rhythm in the suprachiasmatic nucleus (SCN) encoded by the DNA having a sequence described in SEQ ID NO: 3 or by DNA that hybridizes with the DNA described in SEQ ID NO: 3,
(7) a protein involved in the formation of circadian rhythm in the suprachiasmatic nucleus (SCN) encoded by the DNA having a sequence described in SEQ ID NO: 4 or by DNA that hybridizes with the DNA described in SEQ ID NO: 4,
(8) DNA encoding any of the proteins of (1) to (5),
(9) DNA having the sequence described in SEQ ID NO: 3 or DNA that hybridizes with the DNA having the sequence described in SEQ ID NO: 3, wherein the DNA encodes a protein involved in the formation of circadian rhythm in the suprachiasmatic nucleus (SCN),
(10) DNA having the sequence described in SEQ ID NO: 4 or DNA that hybridizes with the DNA having the sequence described in SEQ ID NO: 4, wherein the DNA encodes a protein involved in the formation of circadian rhythm in the suprachiasmatic nucleus (SCN),
(11) a vector carrying any of the DNA of (8) to (10),
(12) a transformant expressibly retaining any of the DNA of (8) to (10), and
(13) a method for producing any of the proteins of (1) to (7), the method comprising culturing the transformant of (12).

Herein, the "circadian periods" means the activity rhythms with a period of approximately 24 hours which are observed in a wide variety of behaviors such as endocrine secretions and body temperature, blood pressure, sleep-wakefulness, and others of an organism.

The expression of the protein of the present invention oscillates autonomously with a circadian period in the suprachiasmatic nucleus (SCN), which is a major circadian pacemaker of the mammalian brain (Moor, R. Y. and Eichler, V. B., Brain Res. 42: 201-206: 1972; Stephan, F. K. and Zucker, I., Proc. Natl. Acad. Sci. USA 69: 1583-1586, 1972). The amino acid sequences of the proteins derived from the human and the mouse included in the present invention are shown in SEQ ID NO: 1 and SEQ ID NO: 2, respectively. The amino acid sequences of these two mammalian proteins fairly homologous with that of the *Drosophila* protein (the period gene product) (Citri, Y., et al., Nature 326: 42-47, 1987). The period gene is required for the expression of the circadian rhythms of locomotive activities and hatching behavior in *Drosophila* (Konopka, R. J. and Benzer, S., Proc. Natl. Acad. Sci. USA 68: 2112-2116, 1971). The oscillations of its mRNA and protein levels in the fly brain are thought to determine the rhythms (Hardin, P. E., et al., Nature 343: 536-540, 1990; Zerr, D. M., et al., J. Neurosci. 10: 2749-2762, 1990). These two proteins show highly homologous with the *Drosophila* protein in the PAS domains which have been suggested to be structurally and functionally important based on the genetic and biochemical studies (Baylies, M. K. et al., Nature 326: 390-392, 1987; Saez, L. and Young, M. W., Neuron 17: 911-920, 1996).

Recently King et al. have cloned the mammalian "Clock" gene, which encodes a bHLH-PAS-polyQ polypeptide (King, D. P., et al., Cell 89: 641-653, 1997; Antoch, M. P., et al., Cell 89: 655-667, 1997). The proteins of the present invention can form dimers with other molecules such as "CLOCK" by means of the PAS-PAS interaction in the circadian clock system.

The proteins of the present invention can be prepared as a recombinant protein utilizing the genetic recombinant technology, or as a natural protein. A recombinant protein can be prepared by culturing the cells transformed with DNA encoding the protein of the present invention as described later. A natural protein can be isolated, for example, from the somatic cell tissues, such as brain, pancreas, kidney, skeletal muscle, liver, lung, placenta, heart, spleen, and testis using an affinity column with an appropriate carrier bound to an antibody that is prepared using the above-mentioned recombinant protein of the present invention.

It is possible for a person skilled in the art to prepare a protein substantially identical to the protein described in SEQ ID NO: 1 or SEQ ID NO: 2 by making amino acid substitutions and other modifications to the protein described in SEQ ID NO: 1 using known methods. Mutations of amino acids in a protein may also occur spotaneously. Thus, the present invention includes modified proteins that result from the modification of amino acids of the protein described in SEQ ID No: 1 or 2 by substitution, deletion, or addition, and are involved in the formation of circadian rhythms in the suprachiasmatic nucleus (SCN). The known methods to modify amino acids include the ODA (Oligonucleotide-directed Dual Amber)-LA PCR method (Hashimoto-Gotoh, T., et al., Gene 152: 271-275, 1995). The amino acids to be substituted are usually within 10 amino acids, preferably within 6 amino acids, and more preferably within 3 amino acids.

It is routine for one skilled in the art to obtain proteins that are substantially functionally equivalent to the protein described in SEQ ID NO: 1 or 2 from DNAs that are highly homologous with the DNA having a sequence described in SEQ ID NO: 3 or 4 and isolated from other organisms using such methods as the known hybridization technique (Church, G. M. and Gilbert, W., Proc. Natl. Acad. Sci. USA 81: 1991-1995, 1984; Sambrook, J., et al., Molecular Cloning, 2^{nd} ed., 1989) based on the DNA sequence described in SEQ ID NO: 3 or 4 (or part thereof). Thus the proteins encoded by the DNA that hybridizes with the DNA sequence described in SEQ ID NO: 3 or 4, which are involved in the formation of circadian rhythms in the suprachiasmatic nucleus (SCN), are also included in the proteins of the present invention. The source of the DNA for hybridization includes mammals such as rats, dogs, cats, monkeys, whales, cattle, pigs, and horses. The DNA encoding the proteins from these other organisms should usually highly homologous with the DNA described in SEQ ID NO: 3 or 4. "Being highly homologous" means having at least 60%, preferably at least 70%, more preferably at least 80%, and still more preferably at least 90% of sequence identity with the DNA described in SEQ ID NO: 3 or 4. The hybridization for isolating such DNAs can be performed, for example, in a mixture consisting of 6 x SSPE, 5 x Denhardt's solution, 0.5% SDS, 100 µl/ml denatured salmon sperm DNA, and 50% formamide, usually at 42°C, less stringently at 32°C, or more stringently at 65°C.

The present invention also relates to DNAs encoding the proteins of the present invention described above. The DNAs encoding the proteins of the present invention can be cDNA, genomic DNA, or synthetic DNA. The DNAs of the present invention can be utilized, for example, to manufacture the proteins of the present invention as recombinant proteins. Namely, the DNA encoding a protein of the present invention (for example, the DNA described in SEQ ID NO: 3 or 4) is inserted into an appropriate expression vector, appropriate cells are transformed with the vector, the transformants are cultured, and the expressed protein is purified to prepare the proteins of the present invention as recombinant proteins.

The preferred cells used for the production of the recombinant proteins include E. coli, yeast, insect cells, and animal cells. The vectors used to express the recombinant proteins within these cells include the pET system, pAUR system, baculovirus vectors (pBlue Bac, etc.), and the CMV or RSV promoter-driven vectors, etc.

The transfection of the vector into the host cell can be done, for example, by electroporation for E. coli and yeast, and the liposome method for insect cells and animal cells. The lithium acetate method can also be used for yeast.

The recombinant protein can be purified from the transformant, for example, by ion exchange, gel filtration, or anti-Per antibody column chromatography.

The proteins or the DNAs of the present invention are applicable to treat disorders related to circadian rhythms, such as sleep phase delay syndrome, sleep phase progression syndrome, non-circadian sleep-wake syndrome, irregular sleep-wake disorder, and time difference syndrome (so-called jet lag). They are also applicable to the labor and health management of irregular night time workers and to prevention of night poriomania in dementia.

### Brief Description of the Drawings

Figure 1 shows the amino acid sequences within the PAS repeats (arrows) that were used to design the primers for IMS-PCR.
Figure 2 is a photograph showing an electrophoresis image of 3 bp ladder markers that were electrophoresed on a 10% non-denaturing PAGE gel in a non-continuous buffer solution system. A 10 bp DNA ladder (BRL) was electrophoresed on lane M.
Figure 3 is a photograph showing an electrophoresis image of the IMS-PCR product (lanes marked with arrows) that was electrophoresed along with 59 bp, 65 bp, and 68 bp of the 3 bp ladder markers (lanes marked with asterisks).
Figure 4 shows an amino acid sequence comparison among the PERIOD family members. hDIAL, mDIAL, and PERIOD indicate the human, the mouse, and the *Drosophila* version of PERIOD, respectively. Shaded or dotted boxes indicate homologous sequences, and C1 through C6 indicate regions conserved among different *Drosophila* species.

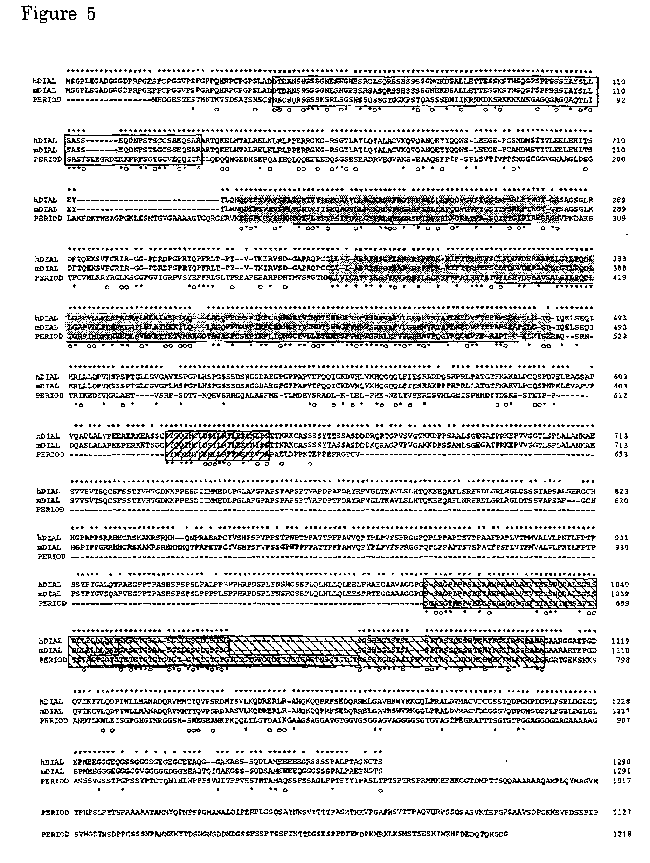

Figure 5 shows an amino acid sequence comparison among the PERIOD family members. hDIAL, mDIAL, and PERIOD indicate the human, the mouse, and the *Drosophila* version of PERIOD, respectively. Shaded or dotted portions indicate homologous sequences. Sequences corresponding to NLS, the PAS-A repeats, the PAS-B repeats, and CLD are underlined, and the TG repeats (the SG repeats in the human and mouse PER) are boxed. Amino acid identities between the human PERIOD and the mouse PERIOD are indicated by asterisks above the human PERIOD sequence. The identities and homologies between the mammalian PERIOD and the *Drosophila* PERIOD are indicated by asterisks and open circles below the *Drosophila* PERIOD sequence.
Figure 6 is a photograph showing the northern blot analysis of hPER. hPER was bound to the filter as a probe, and then G3PDH was bound as a loading control.
Figure 7 is a photograph showing the northern blot analysis of mPer. mPer was bound to the filter as a probe, and then G3PDH was bound as a loading control.
Figure 8 is a photograph showing the results of *in situ* hybridization of mPer in the mouse brain under the LD (top) and the DD (bottom) conditions. SCN is indicated by arrows. The bar indicates 2 mm.
Figure 9 shows the results of quantification of *in situ* hybridization data under the LD (top) and the DD (bottom) conditions. Each data point is the average ± SEM (n=5). ** indicates significance at the 1% significance level, and * at the 5% significance level, compared with the values at ZT16 and CT16. The white portion of the bar represents the light period, and the black portions the dark periods.
Figure 10 shows the results of the competitive RT-PCR analysis on the mPer mRNA under the LD (top) and the DD (bottom) conditions. ΔmPer indicates a competitive factor for mPer and Δβ-actin indicates a competitive factor for β-actin. The white portion of the bar represents the light period, and the black portions the dark periods.

### Best Mode for Carrying out the Invention

The present invention is illustrated in detail below with reference to the following examples, but is not to be construed as being limited thereto.

### Example 1 Isolation of the mammalian homologues of per

In order to isolate the mammalian homologues of per, the inventors have developed a novel method, intramodule scanning (IMS) -PCR. The principle of the method is based on the fact that in the human genome short stretches of DNA sequences (modules) that encode short polypeptide fragments (motifs) are scattered over long genomic distances. If a sufficient number of "intramodule scanning" primers are used to cover the entire length of a gene, the module can be screened with equal frequencies irrespective of their expression levels.

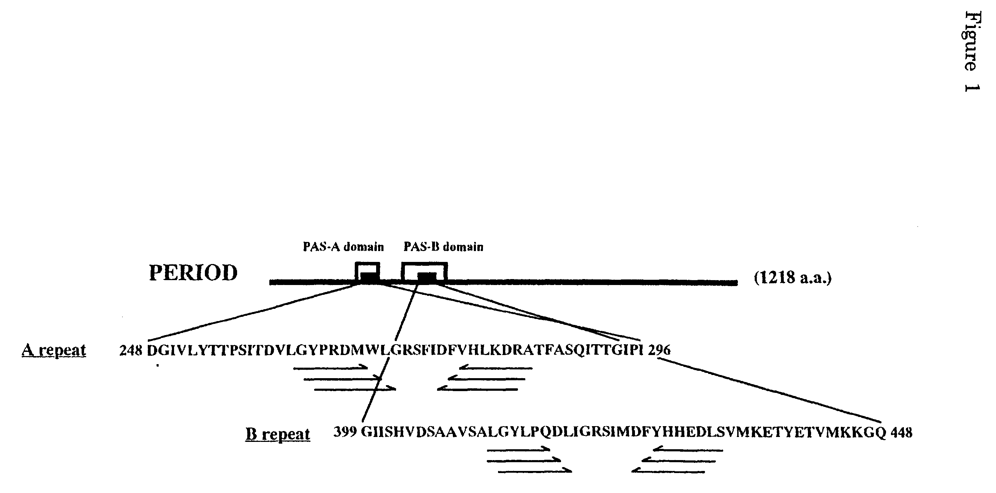

Genetic and biochemical studies have suggested that the PAS domains in dPER are structurally and functionally important (Baylies, M. K. et al., Nature 326: 390-392, 1987; Saez, L. and Young, M. W., Neuron 17: 911-920, 1996). Therefore, we designed 18 different primers corresponding to the internal sequences of the dPER PAS-A and PAS-B repeats (Figure 1). The sequences of the degenerate primer pairs for the PAS-A and PAS-B repeats are as follows:
GTGCTGGGCTACCCN(A/C)GNGA;
CTGGGCTACCCCC(A/G)(A/G)GANATG;
GGCTACCCCC(A/G)(A/G)GANATGTGG;
CTGGGCT(A/T)CCTGCCNCA(A/G);
CTGGGCT(A/T)CCTGCCNCA(A/G)GA;
GGCTACCTGCC(C/T)CA(A/G)GAN(C/T);
GCCCG(G/A)TCCTTCAG(G/A)TGNAC;
TCCTCATG(A/G)TGCAC(A/G)(T/A)ANTC;
ATGTCCTCATG(A/G)TG(C/G)AC(A/G)(A/T)A; and
GACAC(A/G)TCCTCATG(A/G)TG(A/G)TA.
Here, symbols such as A/G mean mixture primers between A and G.

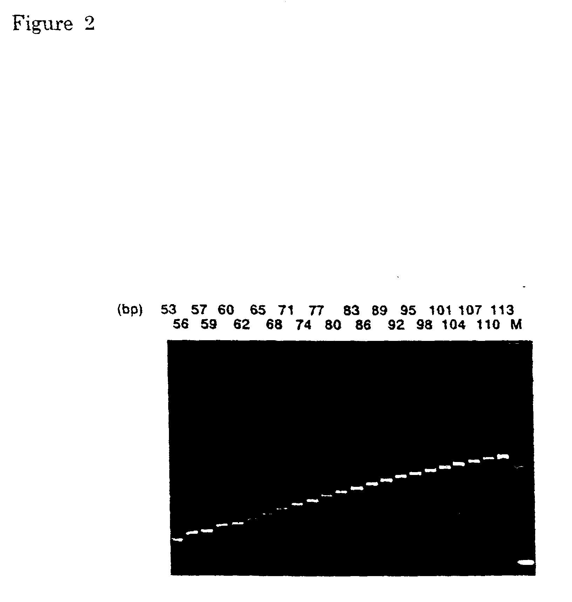

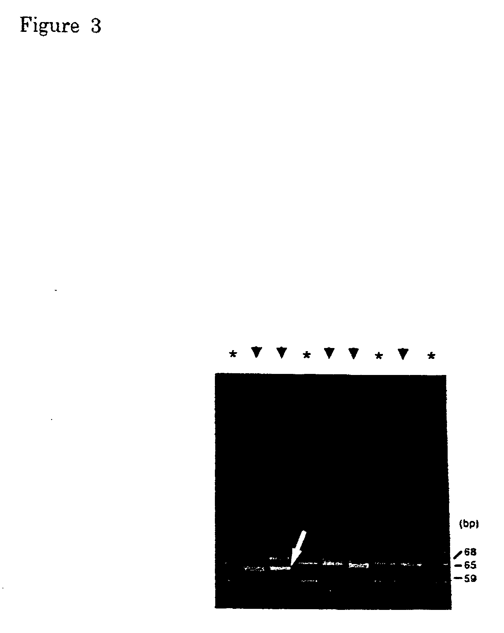

Since homologous polypeptides share common characteristics at the corresponding positions within the molecules, when the corresponding amino acid sequences are used for synthesizing PCR primers, the lengths of the PCR products reflect the characteristics of the domain structure in each polypeptide with respect to the positions. Considering the lengths of a codon (3 bp) and an exon (100 bp on average) in a human gene, we synthesized the 3 bp ladder markers (53 to 113 bp) by PCR using the series of primers and pUC18 as the template. An electrophoretic image of these 3 bp ladder marker and a 10 bp DNA ladder marker (BRL) are shown in Figure 2. The markers were electrophoresed along with the PCR products side by side in a non-continuous buffer solution system (Ito, T., Hohjoh, H. and Sakaki, Y., Electrophoresis 14: 278-282, 1993) on a non-denaturing PAGE (10%) gel (Figure 3).

Each PCR mixture (Sambrook, J., et al., Molecular Cloning, Cold Spring Harbor Laboratory, 1989) contained 0.5 µg of human genomic DNA. The mixture was incubated at 94°C for 1 minute, and subjected to 3 cycles of [94°C for 30 seconds, 37°C for 30 seconds, and 72°C for 30 seconds], followed by 25 cycles of [94°C for 30 seconds, 45°C for 30 seconds, and 72°C for 30 seconds].

The DNA bands of expected lengths were cloned and their sequences determined. Among the 33 clones (59 to 74 bp) derived from the 12 bands that were produced by the nested PCR using a certain primer pair (corresponding to the peptide sequences 5' "GYLPQD" and 3' "FVHHEDI"), the clones of 65 bp were especially amplified 6 to 21 fold. It became clear that the genomic DNA sequence containing the 65 bp fragment has a 106 bp exon encoding 35 amino acid residues that are part of the PAS-B domain consisting of a total of 125 amino acids. We isolated the corresponding cDNA and named human PER (hPER) cDNA. Next, we cloned a mouse homologue (mPer) cDNA using the hPER cDNA as a probe. The nucleotide sequences determined are shown in SEQ ID NO: 3 for hPER, and SEQ ID NO: 4 for mPer. FISH revealed that the hPER gene and the mPer gene were located at 17p12-13.1 and 11B, respectively, which are gene loci in synteny between the two species.

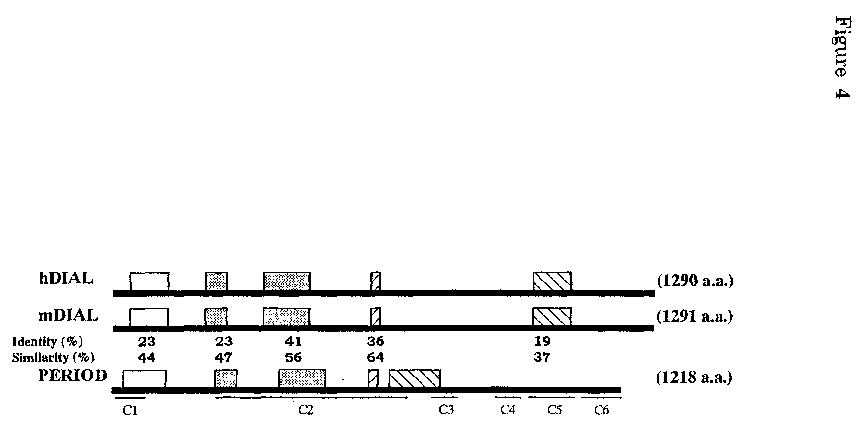

The cDNA sequences of hPER and mPer contain ORF's that are expected to encode 1,290 amino acid residues and 1,291 amino acid residues, respectively. (See Figure 5. The putative amino acid sequence of the hPER gene product is shown in SEQ ID NO: 3, and that of the mPer gene product in SEQ ID NO: 4.) The amino acid identity between hPER and mPER is 92%, clearly indicating that hPER and mPer are conserved between the two species (Figure 5). A homology search using the BLAST program on non-overlapping amino acid databases demonstrated that the two mammalian PER's showed the highest homology with dPER (type A) (Citri, Y., et al., Nature 326:42-47, 1987). Significant homologies between the mammalian PER and the *Drosophila* PER were concentrated on five domains (Figures 4 and 5): I) N-terminal homologous regions (residues 44 to 131 of hPER and mPER); II) PAS-A (residues 217 to 282 for both homologues); III) PAS-B (residues 338 to 456 for both homologues) and its immediate downstream sequence (residues 457 to 485 for both homologues); IV) a short segment corresponding to the downstream region from the site (residue 589) of the per S mutation (which shortens the circadian period) (residues 624 to 645 for both homologues); and V) regions homologous with the PER-C C-terminal region (residues 1006 to 1050 for hPER and residues 1005 to 1049 for mPER), subsequent serine-glycine (SG) repeats (residues 1051 to 1072 for hPER and residues 1050 to 1071 for mPER), and further downstream homologous sequences (residues 1073 to 1108 for hPER and residues 1072 to 1107 for mPER). The homology in these regions are 44%, 47%, 56%, 64%, and 37%, respectively (Figure 4). Although the PAS domains (regions II and III) of the PER homologues are fairly homologous to the corresponding region of dPER, other regions also show high homologies. Five structural domains and functional domains have been identified in dPER: a) the nuclear localization signal (NLS) (residues 66 to 79) (Vosshall, L. B., et al., Science 263: 1606-1609, 1996); b) the PAS domain (residues 233 to 490) necessary for dPER to interact with the NLS of TIM (Saez, L. and Young, M. W., Neuron 17: 911-920, 1996); c) the cytoplasmic localization domain (CLD) (residues 453 to 511) located downstream from the PAS-B repeats (Saez, L. and Young, M. W., Neuron 17: 911-920, 1996); d) the PER-C domain (residues 524 to 685) which interacts with the PAS domain in the self-polypeptide (Huang, Z. J., et al., Science 267: 1169-1172, 1995); and e) the threonine-glycine (TG) repeats (residues 694 to 748) and the immediate downstream region (residues 749 to 868) which control the rhythm of the species-specific mating song of *Drosophila* (Wheeler, D. A., et al., Science 251: 1082-1085, 1991). Thus, NLS, PAS, CLD, the two domains within PER-C, and the TG repeats and a segment next to its C-terminus in each mammalian PER are arranged in exactly the same order as in dPER. Interestingly, the TG repeats of dPER are replaced with short SG repeats in the C-terminal halves of the PER homologues (Figure 5). This segment, which is adjacent to PER-C, and the sequence homologous to the C-terminal side of the TG repeats are located approximately 350 bases downstream from the original locations in dPER (Figure 4). These regions are also highly conserved in both the human and the mouse (Figure 5). Six PER segments (C1-C6) that are highly conserved among different *Drosophila* species are seen (Figure 4) (Colot, H. V., et al., EMBO J. 7: 3929-3937, 1988). Like in the silkmoth homologue of PER, the parts of the mammalian PER that are homologous with dPER are concentrated on the regions corresponding to C1-C3 of dPER (Figure 4) (Reppert, S.M., et al., Neuron 13: 1167-1176, 1994). Considering these observations, hPER and mPer are conclusively the structural homologues of per.

### Example 2 Expression of hPER and mPer

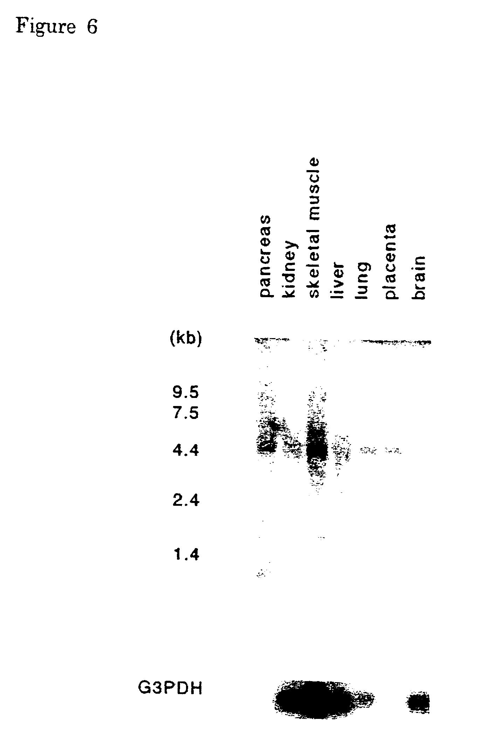

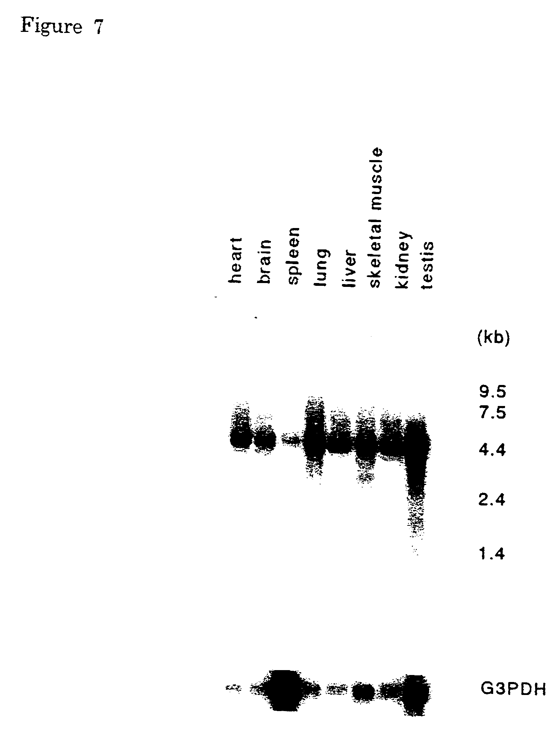

The expression patterns of hPER and mPer were examined by northern hybridization according to the method of Church and Gilbert (Church, G. M. and Gilbert, W., Proc. Natl. Acad. Sci. USA 81: 1991-1995, 1984). The filters were purchased from Clontech. The results are shown in Figure 6 (hPER) and Figure 7 (mPer). The expression product of approximately 4.6 kb was detected in all the tissues tested from the adult human and the mouse. However, the levels of the hPER/mPer transcription product are not uniform as compared with those of glycerol-3-phosphate dehydrogenase (G3PDH), which is an enzyme in the glycolytic pathway and is abundantly and relatively constantly expressed in every cell. The wide distribution of the hPER/mPer expression is not surprising because in *Drosophila* the per expression has been detected in many tissues except the brain (Liu, X., et al., Genes Dev. 2: 228-238, 1988; Saez, L. and Young, M. W., Mol. Cell. Biol. 8: 5378-5385, 1988).

### Example 3 Distribution of the mPer cDNA in the mouse brain

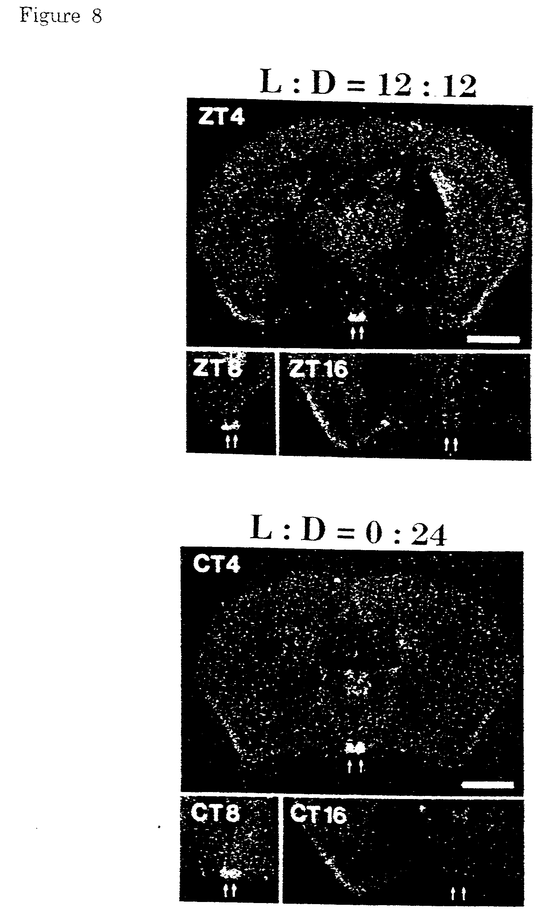

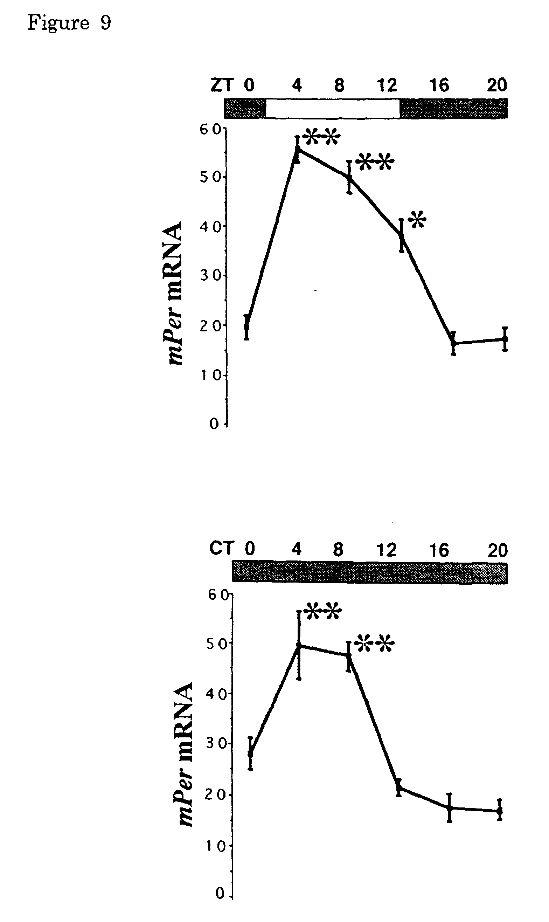

The distribution of the mPer cDNA in the mouse brain was examined by *in situ* hybridization. Continuous cortical sections (40 µm thickness) of the mouse brain were prepared in the cryostat. *In situ* hybridization and determination of mRNA are described in the literature reference (e.g., Ban, Y., Shigeyoshi, Y. and Okamura, H., J. Neurosci. 17: 3920-3931, 1997). The ³³P-labeled probes used in the hybridization were the sense and the antisense strands on the 5' side of the mPer cRNA (nucleotide positions 538-1752; data not shown). After the signals were converted into relative optical concentrations using the ¹⁴C-acrylic acid standard (Amersham, Inc. Plc.), the radioactivity was analyzed on each section on the BioMax film (Kodak) using a microcomputer connected to an image analyzer (MCID, Imaging Research, Inc.). These data were standardized against the difference in signal intensities between the equivalent regions of SCN and corpus callosum. The intensities of optical concentrations in the sections covering from the rostral end to the caudal end of SCN (10 pieces per mouse) were added, and the total was used as the measured value of the mPer mRNA quantity of this region. As a result, weak signals were detected from most brain areas including the cortical structures and non-cortical structures. Stronger mPer mRNA signals were detected from the pyramidal cell layer of piriform cortex, periventricular regions of the caudate putamen, many of the thalamic nuclei, and the granular layer of cerebellar cortex. Surprisingly, the highest mPer expression level in the brain was observed in SCN at a specific time (Figures 8 and 9; explained below).

In order to examine the time dependence of the mPer expression in SCN, mice were synchronized to an environment by keeping them under the 12 h light/12 h dark (LD) conditions. The mPer mRNA was quantified by *in situ* hybridization and the competitive RT-PCR method. The competitive RT-PCR was performed as follows. First, we prepared mouse brain sections (0.5 mm thickness) in the "Mouse Brain Matrix" (Neuroscience, Inc., Tokyo). Using a microdissection needle (600 µm diameter), SCN was pressed out laterally symmetrically from the frozen sections under a stereoscopic microscope. Total RNA was extracted from SCN (n=4) using TRIZOL solution (BRL), treated with DNase I (Stratagene), and purified using TRIZOL LS solution (BRL). "SUPERSCRIPT Preamplification System" (BRL) was used to reverse-transcribe approximately 1 µg of RNA, and the cDNAs of mPer and β-actin were quantified by the competitive PCR method. The PCR products were electrophoresed on a non-denaturing PAGE gel (5.5%), stained with "SYBR Green" (Molecular Probes), and the DNA in appropriate bands was quantified with "FMBI011 fluoroimage analyzer" (Hitachi). The competitive DNA fragments for mPer and β-actin were constructed by making internal deletions in the respective cDNAs. mPer, mPer competitive factor, β-actin, and β-actin competitive factor were 482 bp, 246 bp, 1228 bp, and 1044 bp, respectively.

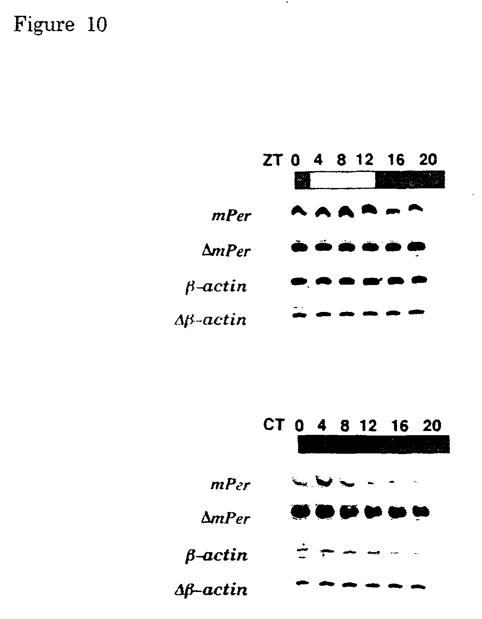

These two methods (*in situ* hybridization and the competitive RT-PCR method) produced similar oscillation profiles in LD (Figures 8 and 10; upper panels). The mPer mRNA quantity reached a peak in the light condition (from ZT4 to ZT8; ZT indicates the time under the LD condition as in Figures 8 to 10), and fell to a minimum in the dark condition (from ZT16 to ZT20) (Figure 9; upper panel). Moreover, under the constant dark condition (DD), there were free-run changes (Figures 8 and 10; lower panels), in which the mPer mRNA levels reached a peak between CT4 and CT8 (CT indicates the time under the DD condition as in Figures 8 to 10) and fell to a minimum between CT16 and CT20 (Figure 9; lower panel). The mPer mRNA in SCN is expressed with a strong and autonomous circadian period under the constant dark condition as described above, suggesting that this gene functions as a circadian rhythm pacemaker. Changes of the mPer mRNA in SCN with a circadian rhythm resemble the nervous activities in this brain region (Inouye, S-T. and Kawamura, H., Proc. Natl. Acad. Sci. USA 76: 5962-5966, 1979; Schwarts, W. J. and Gainer, H., Science 197: 1089-1092, 1977; Gillette, M. U. and Reppert, S. M., Brain Res. Bull. 19: 135-139, 1987), reaching a peak in the daytime and falling to a minimum during the night. mPer may function as a controlling factor of the nervous activities in SCN.

### Industrial Applicability

The present invention provides novel mammalian proteins and their genes involved in the circadian period. The proteins and the DNAs of the present invention are expected to be able to correct abnormalities of the circadian rhythm in the mammals, and would thus be useful for treating disorders related to circadian rhythms, such as sleep phase delay syndrome, sleep phase progression syndrome, non-circadian sleep-wake syndrome, irregular sleep-wake disorder, and time difference syndrome (so-called jet lag). They are also applicable to the labor and health management of irregular night time workers and to the prevention of such disorders as night poriomania in dementia.

## Claims

1. A protein derived from a mammal whose expression level in the suprachiasmatic nucleus (SCN) fluctuates with a circadian period.

2. A protein of claim 1, wherein the mammal is a human.

3. A protein of claim 1, wherein the mammal is a mouse.

4. A protein involved in the formation of circadian rhythm in the suprachiasmatic nucleus (SCN) comprising the amino acid sequence described in SEQ ID NO: 1 or said sequence in which one or more amino acids are substituted, deleted, or added.

5. A protein involved in the formation of circadian rhythm in the suprachiasmatic nucleus (SCN) comprising the amino acid sequence described in SEQ ID NO: 2 or said sequence in which one or more amino acids are substituted, deleted, or added.

6. A protein involved in the formation of circadian rhythm in the suprachiasmatic nucleus (SCN) encoded by the DNA having a sequence described in SEQ ID NO: 3 or by DNA that hybridizes with the DNA described in SEQ ID NO: 3.

7. A protein involved in the formation of circadian rhythm in the suprachiasmatic nucleus (SCN) encoded by the DNA having a sequence described in SEQ ID NO: 4 or by DNA that hybridizes with the DNA described in SEQ ID NO: 4.

8. DNA encoding the protein of any one of claims 1 to 5.

9. DNA having the sequence described in SEQ ID NO: 3 or DNA that hybridizes with the DNA having the sequence described in SEQ ID NO: 3, wherein the DNA encodes a protein involved in the formation of circadian rhythm in the suprachiasmatic nucleus (SCN).

10. DNA having the sequence described in SEQ ID NO: 4 or DNA that hybridizes with the DNA having the sequence described in SEQ ID NO: 4, wherein the DNA encodes a protein involved in the formation of circadian rhythm in the suprachiasmatic nucleus (SCN).

11. A vector carrying the DNA of any one of claims 8 to 10.

12. A transformant expressibly retaining the DNA of any one of claims 8 to 10.

13. A method for producing the protein of any one of claims 1 to 7, the method comprising culturing the transformant of claim 12.


SEQUENCE LISTING

<110> SAKAKI, Yoshiyuki

<120> Mammalian Genes Involved in Circadian Periods

<130> SEN-903PCT

<150> JP 9-267846
<151> 1997-09-12

<160> 4

<210> 1
<211> 1290
<212> PRT
<213> Homo sapiens

<400> 1
Met Ser Gly Pro Leu Glu Gly Ala Asp Gly Gly Gly Asp Pro Arg Pro
1               5                   10                  15

Gly Glu Ser Phe Cys Pro Gly Gly Val Pro Ser Pro Gly Pro Pro Gln
            20                  25                  30

His Arg Pro Cys Pro Gly Pro Ser Leu Ala Asp Asp Thr Asp Ala Asn
        35                  40                  45

Ser Asn Gly Ser Ser Gly Asn Glu Ser Asn Gly His Glu Ser Arg Gly
    50                  55                  60

Ala Ser Gln Arg Ser Ser His Ser Ser Ser Ser Gly Asn Gly Lys Asp
65                  70                  75                  80

Ser Ala Leu Leu Glu Thr Thr Glu Ser Ser Lys Ser Thr Asn Ser Gln
                85                  90                  95

Ser Pro Ser Pro Pro Ser Ser Ser Ile Ala Tyr Ser Leu Leu Ser Ala

            100                 105                 110

Ser Ser Glu Gln Asp Asn Pro Ser Thr Ser Gly Cys Ser Ser Glu Gln
        115                 120                 125

Ser Ala Arg Ala Arg Thr Gln Lys Glu Leu Met Thr Ala Leu Arg Glu
    130                 135                 140

Leu Lys Leu Arg Leu Pro Pro Glu Arg Arg Gly Lys Gly Arg Ser Gly
145                 150                 155                 160

Thr Leu Ala Thr Leu Gln Tyr Ala Leu Ala Cys Val Lys Gln Val Gln
                165                 170                 175

Ala Asn Gln Glu Tyr Tyr Gln Gln Trp Ser Leu Glu Glu Gly Glu Pro
            180                 185                 190

Cys Ser Met Asp Met Ser Thr Tyr Thr Leu Glu Glu Leu Glu His Ile
        195                 200                 205

Thr Ser Glu Tyr Thr Leu Gln Asn Gln Asp Thr Phe Ser Val Ala Val
    210                 215                 220

Ser Phe Leu Thr Gly Arg Ile Val Tyr Ile Ser Glu Gln Ala Ala Val
225                 230                 235                 240

Leu Leu Arg Cys Lys Arg Asp Val Phe Arg Gly Thr Arg Phe Ser Glu
                245                 250                 255

Leu Leu Ala Pro Gln Asp Val Gly Val Phe Tyr Gly Ser Thr Ala Pro
            260                 265                 270

Ser Arg Leu Pro Thr Trp Gly Thr Gly Ala Ser Ala Gly Ser Gly Leu
        275                 280                 285

Arg Asp Phe Thr Gln Glu Lys Ser Val Phe Cys Arg Ile Arg Gly Gly
    290                 295                 300

Pro Asp Arg Asp Pro Gly Pro Arg Tyr Gln Pro Phe Arg Leu Thr Pro
305                 310                 315                 320

Tyr Val Thr Lys Ile Arg Val Ser Asp Gly Ala Pro Ala Gln Pro Cys
                325                 330                 335

Cys Leu Leu Ile Ala Glu Arg Ile His Ser Gly Tyr Glu Ala Pro Arg
            340                 345                 350

Ile Pro Pro Asp Lys Arg Ile Phe Thr Thr Arg His Thr Pro Ser Cys
        355                 360                 365

Leu Phe Gln Asp Val Asp Glu Arg Ala Ala Pro Leu Leu Gly Tyr Leu
    370                 375                 380

Pro Gln Asp Leu Leu Gly Ala Pro Val Leu Leu Phe Leu His Pro Glu
385                 390                 395                 400

Asp Arg Pro Leu Met Leu Ala Ile His Lys Lys Ile Leu Gln Leu Ala
                405                 410                 415

Gly Gln Pro Phe Asp His Ser Pro Ile Arg Phe Cys Ala Arg Asn Gly
            420                 425                 430

Glu Tyr Val Thr Met Asp Thr Ser Trp Ala Gly Phe Val His Pro Trp
        435                 440                 445

Ser Arg Lys Val Ala Phe Val Leu Gly Arg His Lys Val Arg Thr Ala
    450                 455                 460

Pro Leu Asn Glu Asp Val Phe Thr Pro Pro Ala Pro Ser Pro Ala Pro
465                 470                 475                 480

Ser Leu Asp Thr Asp Ile Gln Glu Leu Ser Glu Gln Ile His Arg Leu
                485                 490                 495

Leu Leu Gln Pro Val His Ser Pro Ser Pro Thr Gly Leu Cys Gly Val
            500                 505                 510

Gly Ala Val Thr Ser Pro Gly Pro Leu His Ser Pro Gly Ser Ser Ser
        515                 520                 525

Asp Ser Asn Gly Gly Asp Ala Glu Gly Pro Gly Pro Pro Ala Pro Val
    530                 535                 540

Thr Phe Gln Gln Ile Cys Lys Asp Val His Leu Val Lys His Gln Gly
545                 550                 555                 560

Gln Gln Leu Phe Ile Glu Ser Arg Ala Arg Pro Gln Ser Arg Pro Arg
                565                 570                 575

Leu Pro Ala Thr Gly Thr Phe Lys Ala Lys Ala Leu Pro Cys Gln Ser
            580                 585                 590

Pro Asp Pro Glu Leu Glu Ala Gly Ser Ala Pro Val Gln Ala Pro Leu
        595                 600                 605

Ala Leu Val Pro Glu Glu Ala Glu Arg Lys Glu Ala Ser Ser Cys Ser
    610                 615                 620

Tyr Gln Gln Ile Asn Cys Leu Asp Ser Ile Leu Arg Tyr Leu Glu Ser
625                 630                 635                 640

Cys Asn Leu Pro Ser Thr Thr Lys Arg Lys Cys Ala Ser Ser Ser Ser
                645                 650                 655

Tyr Thr Thr Ser Ser Ala Ser Asp Asp Asp Arg Gln Arg Thr Gly Pro
            660                 665                 670

Val Ser Val Gly Thr Lys Lys Asp Pro Pro Ser Ala Ala Leu Ser Gly
        675                 680                 685

Glu Gly Ala Thr Pro Arg Lys Glu Pro Val Val Gly Gly Thr Leu Ser
    690                 695                 700

Pro Leu Ala Leu Ala Asn Lys Ala Glu Ser Val Val Ser Val Thr Ser

705                 710                 715                 720

Gln Cys Ser Phe Ser Ser Thr Ile Val His Val Gly Asp Lys Lys Pro
                725                 730                 735

Pro Glu Ser Asp Ile Ile Met Met Glu Asp Leu Pro Gly Leu Ala Pro
            740                 745                 750

Gly Pro Ala Pro Ser Pro Ala Pro Ser Pro Thr Val Ala Pro Asp Pro
        755                 760                 765

Ala Pro Asp Ala Tyr Arg Pro Val Gly Leu Thr Lys Ala Val Leu Ser
    770                 775                 780

Leu His Thr Gln Lys Glu Glu Gln Ala Phe Leu Ser Arg Phe Arg Asp
785                 790                 795                 800

Leu Gly Arg Leu Arg Gly Leu Asp Ser Ser Ser Thr Ala Pro Ser Ala
                805                 810                 815

Leu Gly Glu Arg Gly Cys His His Gly Pro Ala Pro Pro Ser Arg Arg
            820                 825                 830

His His Cys Arg Ser Lys Ala Lys Arg Ser Arg His His Gln Asn Pro
        835                 840                 845

Arg Ala Glu Ala Pro Cys Tyr Val Ser His Pro Ser Pro Val Pro Pro
    850                 855                 860

Ser Thr Pro Trp Pro Thr Pro Pro Ala Thr Thr Pro Phe Pro Ala Val
865                 870                 875                 880

Val Gln Pro Tyr Pro Leu Pro Val Phe Ser Pro Arg Gly Gly Pro Gln
                885                 890                 895

Pro Leu Pro Pro Ala Pro Thr Ser Val Pro Pro Ala Ala Phe Pro Ala
            900                 905                 910

Pro Leu Val Thr Pro Met Val Ala Leu Val Leu Pro Asn Tyr Leu Phe
        915                 920                 925

Pro Thr Pro Ser Ser Tyr Pro Tyr Gly Ala Leu Gln Thr Pro Ala Glu
    930                 935                 940

Gly Pro Pro Thr Pro Ala Ser His Ser Pro Ser Pro Ser Leu Pro Ala
945                 950                 955                 960

Leu Pro Pro Ser Pro Pro His Arg Pro Asp Ser Pro Leu Phe Asn Ser
                965                 970                 975

Arg Cys Ser Ser Pro Leu Gln Leu Asn Leu Leu Gln Leu Glu Glu Leu
            980                 985                 990

Pro Arg Ala Glu Gly Ala Ala Val Ala Gly Gly Pro Gly Ser Ser Ala
        995                 1000                1005

Gly Pro Pro Pro Pro Ser Ala Glu Ala Ala Glu Pro Glu Ala Arg Leu
    1010                1015                1020

Ala Glu Val Thr Glu Ser Ser Asn Gln Asp Ala Leu Ser Gly Ser Ser
1025                1030                1035                1040

Asp Leu Leu Glu Leu Leu Leu Gln Glu Asp Ser Arg Ser Gly Thr Gly
                1045                1050                1055

Ser Ala Ala Ser Gly Ser Leu Gly Ser Gly Leu Gly Ser Gly Ser Gly
            1060                1065                1070

Ser Gly Ser His Glu Gly Gly Ser Thr Ser Ala Ser Ile Thr Arg Ser
        1075                1080                1085

Ser Gln Ser Ser His Thr Ser Lys Tyr Phe Gly Ser Ile Asp Ser Ser
    1090                1095                1100

Glu Ala Glu Ala Gly Ala Ala Arg Gly Gly Ala Glu Pro Gly Asp Gln
1105                1110                1115                1120

Val Ile Lys Tyr Val Leu Gln Asp Pro Ile Trp Leu Leu Met Ala Asn
                1125                1130                1135

Ala Asp Gln Arg Val Met Met Thr Tyr Gln Val Pro Ser Arg Asp Met
            1140                1145                1150

Thr Ser Val Leu Lys Gln Asp Arg Glu Arg Leu Arg Ala Met Gln Lys
        1155                1160                1165

Gln Gln Pro Arg Phe Ser Glu Asp Gln Arg Arg Glu Leu Gly Ala Val
    1170                1175                1180

His Ser Trp Val Arg Lys Gly Gln Leu Pro Arg Ala Leu Asp Val Met
1185                1190                1195                1200

Ala Cys Val Asp Cys Gly Ser Ser Thr Gln Asp Pro Gly His Pro Asp
                1205                1210                1215

Asp Pro Leu Phe Ser Glu Leu Asp Gly Leu Gly Leu Glu Pro Met Glu
            1220                1225                1230

Glu Gly Gly Gly Glu Gln Gly Ser Ser Gly Gly Gly Ser Gly Glu Gly
        1235                1240                1245

Glu Gly Cys Glu Glu Ala Gln Gly Gly Ala Lys Ala Ser Ser Ser Gln
    1250                1255                1260

Asp Leu Ala Met Glu Glu Glu Glu Glu Gly Arg Ser Ser Ser Ser Pro
1265                1270                1275                1280

Ala Leu Pro Thr Ala Gly Asn Cys Thr Ser
                1285                1290

<210> 2
<211> 1291
<212> PRT
<213> Mus musculus

<400> 2
Met Ser Gly Pro Leu Glu Gly Ala Asp Gly Gly Gly Asp Pro Arg Pro
1               5                   10                  15

Gly Glu Pro Phe Cys Pro Gly Gly Val Pro Ser Pro Gly Ala Pro Gln
            20                  25                  30

His Arg Pro Cys Pro Gly Pro Ser Leu Ala Asp Asp Thr Asp Ala Asn
        35                  40                  45

Ser Asn Gly Ser Ser Gly Asn Glu Ser Asn Gly Pro Glu Ser Arg Gly
    50                  55                  60

Ala Ser Gln Arg Ser Ser His Ser Ser Ser Ser Gly Asn Gly Lys Asp
65                  70                  75                  80

Ser Ala Leu Leu Glu Thr Thr Glu Ser Ser Lys Ser Thr Asn Ser Gln
                85                  90                  95

Ser Pro Ser Pro Pro Ser Ser Ser Ile Ala Tyr Ser Leu Leu Ser Ala
            100                 105                 110

Ser Ser Glu Gln Asp Asn Pro Ser Thr Ser Gly Cys Ser Ser Glu Gln
        115                 120                 125

Ser Ala Arg Ala Arg Thr Gln Lys Glu Leu Met Thr Ala Leu Arg Glu
    130                 135                 140

Leu Lys Leu Arg Leu Pro Pro Glu Arg Arg Gly Lys Gly Arg Ser Gly
145                 150                 155                 160

Thr Leu Ala Thr Leu Gln Tyr Ala Leu Ala Cys Val Lys Gln Val Gln
                165                 170                 175

Ala Asn Gln Glu Tyr Tyr Gln Gln Trp Ser Leu Glu Glu Gly Glu Pro
            180                 185                 190

Cys Ala Met Asp Met Ser Thr Tyr Thr Leu Glu Glu Leu Glu His Ile
        195                 200                 205

Thr Ser Glu Tyr Thr Leu Arg Asn Gln Asp Thr Phe Ser Val Ala Val
    210                 215                 220

Ser Phe Leu Thr Gly Arg Ile Val Tyr Ile Ser Glu Gln Ala Gly Val
225                 230                 235                 240

Leu Leu Arg Cys Lys Arg Asp Val Phe Arg Gly Ala Arg Phe Ser Glu
                245                 250                 255

Leu Leu Ala Pro Gln Asp Val Gly Val Phe Tyr Gly Ser Thr Thr Pro
            260                 265                 270

Ser Arg Leu Pro Thr Trp Gly Thr Gly Thr Ser Ala Gly Ser Gly Leu
        275                 280                 285

Lys Asp Phe Thr Gln Glu Lys Ser Val Phe Cys Arg Ile Arg Gly Gly
    290                 295                 300

Pro Asp Arg Asp Pro Gly Pro Arg Tyr Gln Pro Phe Arg Leu Thr Pro
305                 310                 315                 320

Tyr Val Thr Lys Ile Arg Val Ser Asp Gly Ala Pro Ala Gln Pro Cys
                325                 330                 335

Cys Leu Leu Ile Ala Glu Arg Ile His Ser Gly Tyr Glu Ala Pro Arg
            340                 345                 350

Ile Pro Pro Asp Lys Arg Ile Phe Thr Thr Arg His Thr Pro Ser Cys
        355                 360                 365

Leu Phe Gln Asp Val Asp Glu Arg Ala Ala Pro Leu Leu Gly Tyr Leu
    370                 375                 380

Pro Gln Asp Leu Leu Gly Ala Pro Val Leu Leu Phe Leu His Pro Glu
385                 390                 395                 400

Asp Arg Pro Leu Met Leu Ala Ile His Lys Lys Ile Leu Gln Leu Ala
                405                 410                 415

Gly Gln Pro Phe Asp His Ser Pro Ile Arg Phe Cys Ala Arg Asn Gly
            420                 425                 430

Glu Tyr Val Thr Met Asp Thr Ser Trp Ala Gly Phe Val His Pro Trp
        435                 440                 445

Ser Arg Lys Val Ala Phe Val Leu Gly Arg His Lys Val Arg Thr Ala
    450                 455                 460

Pro Leu Asn Glu Asp Val Phe Thr Pro Pro Ala Pro Ser Pro Ala Pro
465                 470                 475                 480

Ser Leu Asp Ser Asp Ile Gln Glu Leu Ser Glu Gln Ile His Arg Leu
                485                 490                 495

Leu Leu Gln Pro Val His Ser Ser Ser Pro Thr Gly Leu Cys Gly Val
            500                 505                 510

Gly Pro Leu Met Ser Pro Gly Pro Leu His Ser Pro Gly Ser Ser Ser
        515                 520                 525

Asp Ser Asn Gly Gly Asp Ala Glu Gly Pro Gly Pro Pro Ala Pro Val
    530                 535                 540

Thr Phe Gln Gln Ile Cys Lys Asp Val His Leu Val Lys His Gln Gly
545                 550                 555                 560

Gln Gln Leu Phe Ile Glu Ser Arg Ala Lys Pro Pro Pro Arg Pro Arg
                565                 570                 575

Leu Leu Ala Thr Gly Thr Phe Lys Ala Lys Val Leu Pro Cys Gln Ser
            580                 585                 590

Pro Asn Pro Glu Leu Glu Val Ala Pro Val Pro Asp Gln Ala Ser Leu

        595                 600                 605

Ala Leu Ala Pro Glu Glu Pro Glu Arg Lys Glu Thr Ser Gly Cys Ser
    610                 615                 620

Tyr Gln Gln Ile Asn Cys Leu Asp Ser Ile Leu Arg Tyr Leu Glu Ser
625                 630                 635                 640

Cys Asn Ile Pro Ser Thr Thr Lys Arg Lys Cys Ala Ser Ser Ser Ser
                645                 650                 655

Tyr Thr Ala Ser Ser Ala Ser Asp Asp Asp Lys Gln Arg Ala Gly Pro
            660                 665                 670

Val Pro Val Gly Ala Lys Lys Asp Pro Ser Ser Ala Met Leu Ser Gly
        675                 680                 685

Glu Gly Ala Thr Pro Arg Lys Glu Pro Val Val Gly Gly Thr Leu Ser
    690                 695                 700

Pro Leu Ala Leu Ala Asn Lys Ala Glu Ser Val Val Ser Val Thr Ser
705                 710                 715                 720

Gln Cys Ser Phe Ser Ser Thr Ile Val His Val Gly Asp Lys Lys Pro
                725                 730                 735

Pro Glu Ser Asp Ile Ile Met Met Glu Asp Leu Pro Gly Leu Ala Pro
            740                 745                 750

Gly Pro Ala Pro Ser Pro Ala Pro Ser Pro Thr Val Ala Pro Asp Pro
        755                 760                 765

Thr Pro Asp Ala Tyr Arg Pro Val Gly Leu Thr Lys Ala Val Leu Ser
    770                 775                 780

Leu His Thr Gln Lys Glu Glu Gln Ala Phe Leu Asn Arg Phe Arg Asp
785                 790                 795                 800

Leu Gly Arg Leu Arg Gly Leu Asp Thr Ser Ser Val Ala Pro Ser Ala
                805                 810                 815

Pro Gly Cys His His Gly Pro Ile Pro Pro Gly Arg Arg His His Cys
            820                 825                 830

Arg Ser Lys Ala Lys Arg Ser Arg His His His His Gln Thr Pro Arg
        835                 840                 845

Pro Glu Thr Pro Cys Tyr Val Ser His Pro Ser Pro Val Pro Ser Ser
    850                 855                 860

Gly Pro Trp Pro Pro Pro Pro Ala Thr Thr Pro Phe Pro Ala Met Val
865                 870                 875                 880

Gln Pro Tyr Pro Leu Pro Val Phe Ser Pro Arg Gly Gly Pro Gln Pro
                885                 890                 895

Leu Pro Pro Ala Pro Thr Ser Val Ser Pro Ala Thr Phe Pro Ser Pro
            900                 905                 910

Leu Val Thr Pro Met Val Ala Leu Val Leu Pro Asn Tyr Leu Phe Pro
        915                 920                 925

Thr Pro Pro Ser Tyr Pro Tyr Gly Val Ser Gln Ala Pro Val Glu Gly
    930                 935                 940

Pro Pro Thr Pro Ala Ser His Ser Pro Ser Pro Ser Leu Pro Pro Pro
945                 950                 955                 960

Pro Leu Ser Pro Pro His Arg Pro Asp Ser Pro Leu Phe Asn Ser Arg
                965                 970                 975

Cys Ser Ser Pro Leu Gln Leu Asn Leu Leu Gln Leu Glu Glu Ser Pro
            980                 985                 990

Arg Thr Glu Gly Gly Ala Ala Ala Gly Gly Pro Gly Ser Ser Ala Gly
        995                 1000                1005

Pro Leu Pro Pro Ser Glu Glu Thr Ala Glu Pro Glu Ala Arg Leu Val
    1010                1015                1020

Glu Val Thr Glu Ser Ser Asn Gln Asp Ala Leu Ser Gly Ser Ser Asp
1025                1030                1035                1040

Leu Leu Glu Leu Leu Leu Gln Glu Asp Ser Arg Ser Gly Thr Gly Ser
                1045                1050                1055

Ala Ala Ser Gly Ser Leu Gly Ser Gly Leu Gly Ser Gly Ser Gly Ser
            1060                1065                1070

Gly Ser His Glu Gly Gly Ser Thr Ser Ala Ser Ile Thr Arg Ser Ser
        1075                1080                1085

Gln Ser Ser His Thr Ser Lys Tyr Phe Gly Ser Ile Asp Ser Ser Glu
    1090                1095                1100

Ala Glu Ala Gly Ala Ala Arg Ala Arg Thr Glu Pro Gly Asp Gln Val
1105                1110                1115                1120

Ile Lys Cys Val Leu Gln Asp Pro Ile Trp Leu Leu Met Ala Asn Ala
                1125                1130                1135

Asp Gln Arg Val Met Met Thr Tyr Gln Val Pro Ser Arg Asp Ala Ala
            1140                1145                1150

Ser Val Leu Lys Gln Asp Arg Glu Arg Leu Arg Ala Met Gln Lys Gln
        1155                1160                1165

Gln Pro Arg Phe Ser Glu Asp Gln Arg Arg Glu Leu Gly Ala Val His
    1170                1175                1180

Ser Trp Val Arg Lys Gly Gln Leu Pro Arg Ala Leu Asp Val Met Ala
1185                1190                1195                1200

Cys Val Asp Cys Gly Ser Ser Val Gln Asp Pro Gly His Ser Asp Asp

                1205                1210                1215

Pro Leu Phe Ser Glu Leu Asp Gly Leu Gly Leu Glu Pro Met Glu Glu
            1220                1225                1230

Gly Gly Gly Glu Gly Gly Gly Cys Gly Val Gly Gly Gly Gly Gly Asp
        1235                1240                1245

Gly Gly Glu Glu Ala Gln Thr Gln Ile Gly Ala Lys Gly Ser Ser Ser
    1250                1255                1260

Gln Asp Ser Ala Met Glu Glu Glu Glu Gln Gly Gly Gly Ser Ser Ser
1265                1270                1275                1280

Pro Ala Leu Pro Ala Glu Glu Asn Ser Thr Ser
                1285                1290

<210> 3
<211> 3873
<212> DNA
<213> Homo sapiens

<220>
<221> CDS
<222> (1)..(3873)

<400> 3
atg agt ggc ccc cta gaa ggg gct gat ggg gga ggg gac ccc agg cct 48
Met Ser Gly Pro Leu Glu Gly Ala Asp Gly Gly Gly Asp Pro Arg Pro
1               5                   10                  15

ggg gaa tca ttt tgt cct ggg ggc gtc cca tcc cct ggg ccc cca cag 96
Gly Glu Ser Phe Cys Pro Gly Gly Val Pro Ser Pro Gly Pro Pro Gln
            20                  25                  30

cac cgg cct tgc cca ggc ccc agc ctg gcc gat gac acc gat gcc aac 144
His Arg Pro Cys Pro Gly Pro Ser Leu Ala Asp Asp Thr Asp Ala Asn
        35                  40                  45

agc aat ggt tca agt ggc aat gag tcc aac ggg cat gag tct aga ggc 192
Ser Asn Gly Ser Ser Gly Asn Glu Ser Asn Gly His Glu Ser Arg Gly
    50                  55                  60

gca tct cag cgg agc tca cac agc tcc tcc tca ggc aac ggc aag gac 240
Ala Ser Gln Arg Ser Ser His Ser Ser Ser Ser Gly Asn Gly Lys Asp
65                  70                  75                  80

tca gcc ctg ctg gag acc act gag agc agc aag agc aca aac tct cag 288
Ser Ala Leu Leu Glu Thr Thr Glu Ser Ser Lys Ser Thr Asn Ser Gln
                85                  90                  95

agc cca tcc cca ccc agc agt tcc att gcc tac agc ctc ctg agt gcc 336
Ser Pro Ser Pro Pro Ser Ser Ser Ile Ala Tyr Ser Leu Leu Ser Ala
            100                 105                 110

agc tca gag cag gac aac ccg tcc acc agt ggc tgc agc agt gaa cag 384
Ser Ser Glu Gln Asp Asn Pro Ser Thr Ser Gly Cys Ser Ser Glu Gln
        115                 120                 125

tca gcc cgg gca agg act cag aag gaa ctc atg aca gca ctt cga gag 432
Ser Ala Arg Ala Arg Thr Gln Lys Glu Leu Met Thr Ala Leu Arg Glu
    130                 135                 140

ctc aag ctt cga ctg ccg cca gag cgc cgg ggc aag ggc cgc tct ggg 480
Leu Lys Leu Arg Leu Pro Pro Glu Arg Arg Gly Lys Gly Arg Ser Gly
145                 150                 155                 160

acc ctg gcc acg ctg cag tac gca ctg gcc tgt gtc aag cag gtg cag 528
Thr Leu Ala Thr Leu Gln Tyr Ala Leu Ala Cys Val Lys Gln Val Gln
                165                 170                 175

gcc aac cag gaa tac tac cag cag tgg agc ctg gag gag ggc gag cct 576
Ala Asn Gln Glu Tyr Tyr Gln Gln Trp Ser Leu Glu Glu Gly Glu Pro
            180                 185                 190

tgc tcc atg gac atg tcc acc tat acc ctg gag gag ctg gag cac atc 624

Cys Ser Met Asp Met Ser Thr Tyr Thr Leu Glu Glu Leu Glu His Ile
        195                 200                 205

acg tct gag tac aca ctt cag aac cag gat acc ttc tca gtg gct gtc 672
Thr Ser Glu Tyr Thr Leu Gln Asn Gln Asp Thr Phe Ser Val Ala Val
    210                 215                 220

tcc ttc ctg acg ggc cga atc gtc tac att tcg gag cag gca gcc gtc 720
Ser Phe Leu Thr Gly Arg Ile Val Tyr Ile Ser Glu Gln Ala Ala Val
225                 230                 235                 240

ctg ctg cgt tgc aag cgg gac gtg ttc cgg ggt acc cgc ttc tct gag 768
Leu Leu Arg Cys Lys Arg Asp Val Phe Arg Gly Thr Arg Phe Ser Glu
                245                 250                 255

ctc ctg gct ccc cag gat gtg gga gtc ttc tat ggt tcc act gct cca 816
Leu Leu Ala Pro Gln Asp Val Gly Val Phe Tyr Gly Ser Thr Ala Pro
            260                 265                 270

tct cgc ctg ccc acc tgg ggc aca ggg gcc tca gca ggt tca ggc ctc 864
Ser Arg Leu Pro Thr Trp Gly Thr Gly Ala Ser Ala Gly Ser Gly Leu
        275                 280                 285

agg gac ttt acc cag gag aag tcc gtc ttc tgc cgt atc aga gga ggt 912
Arg Asp Phe Thr Gln Glu Lys Ser Val Phe Cys Arg Ile Arg Gly Gly
    290                 295                 300

cct gac cgg gat cca ggg cct cgg tac cag cca ttc cgc cta acc ccg 960
Pro Asp Arg Asp Pro Gly Pro Arg Tyr Gln Pro Phe Arg Leu Thr Pro
305                 310                 315                 320

tat gtg acc aag atc cgg gtc tca gat ggg gcc cct gca cag ccg tgc 1008
Tyr Val Thr Lys Ile Arg Val Ser Asp Gly Ala Pro Ala Gln Pro Cys
                325                 330                 335

tgc ctg ctg att gca gag cgc atc cat tcg ggt tac gaa gct ccc cgg 1056
Cys Leu Leu Ile Ala Glu Arg Ile His Ser Gly Tyr Glu Ala Pro Arg
            340                 345                 350

ata ccc cct gac aag agg att ttc act acg cgg cac aca ccc agc tgc 1104
Ile Pro Pro Asp Lys Arg Ile Phe Thr Thr Arg His Thr Pro Ser Cys
        355                 360                 365

ctc ttc cag gat gtg gat gaa agg gct gcc ccc ctg ctg ggc tac ctg 1152
Leu Phe Gln Asp Val Asp Glu Arg Ala Ala Pro Leu Leu Gly Tyr Leu
    370                 375                 380

ccc cag gac ctc ctg ggg gcc cca gtg ctc ctg ttc ctg cat cct gag 1200
Pro Gln Asp Leu Leu Gly Ala Pro Val Leu Leu Phe Leu His Pro Glu
385                 390                 395                 400

gac cga ccc ctc atg ctg gct atc cac aag aag att ctg cag ttg gcg 1248
Asp Arg Pro Leu Met Leu Ala Ile His Lys Lys Ile Leu Gln Leu Ala
                405                 410                 415

ggc cag ccc ttt gac cac tcc cct atc cgc ttc tgt gcc cgc aac ggg 1296
Gly Gln Pro Phe Asp His Ser Pro Ile Arg Phe Cys Ala Arg Asn Gly
            420                 425                 430

gag tat gtc acc atg gac acc agc tgg gct ggc ttt gtg cac ccc tgg 1344
Glu Tyr Val Thr Met Asp Thr Ser Trp Ala Gly Phe Val His Pro Trp
        435                 440                 445

agc cgc aag gta gcc ttc gtg ttg ggc cgc cac aaa gta cgc acg gcc 1392
Ser Arg Lys Val Ala Phe Val Leu Gly Arg His Lys Val Arg Thr Ala
    450                 455                 460

ccc ctg aat gag gac gtg ttc act ccc ccg gcc ccc agc cca gct ccc 1440
Pro Leu Asn Glu Asp Val Phe Thr Pro Pro Ala Pro Ser Pro Ala Pro
465                 470                 475                 480

tcc ctg gac act gat atc cag gag ctg tca gag cag atc cac cgg ctg 1488
Ser Leu Asp Thr Asp Ile Gln Glu Leu Ser Glu Gln Ile His Arg Leu
                485                 490                 495

ctg ctg cag ccc gtc cac agc ccc agc ccc acg gga ctc tgt gga gtc 1536

Leu Leu Gln Pro Val His Ser Pro Ser Pro Thr Gly Leu Cys Gly Val
            500                 505                 510

ggc gcc gtg aca tcc cca ggc cct ctc cac agc cct ggg tcc tcc agt 1584
Gly Ala Val Thr Ser Pro Gly Pro Leu His Ser Pro Gly Ser Ser Ser
        515                 520                 525

gat agc aac ggg ggt gat gca gag ggg cct ggg cct cct gcg cca gtg 1632
Asp Ser Asn Gly Gly Asp Ala Glu Gly Pro Gly Pro Pro Ala Pro Val
    530                 535                 540

act ttc cag cag atc tgt aag gat gtg cat ctg gtg aag cac cag ggc 1680
Thr Phe Gln Gln Ile Cys Lys Asp Val His Leu Val Lys His Gln Gly
545                 550                 555                 560

cag cag ctt ttt att gag tct cgg gcc cgg cct cag tcc cgg ccc cgc 1728
Gln Gln Leu Phe Ile Glu Ser Arg Ala Arg Pro Gln Ser Arg Pro Arg
                565                 570                 575

ctc cct gct aca ggc acg ttc aag gcc aag gcc ctt ccc tgc caa tcc 1776
Leu Pro Ala Thr Gly Thr Phe Lys Ala Lys Ala Leu Pro Cys Gln Ser
            580                 585                 590

cca gac cca gag ctg gag gcg ggt tct gct ccc gtc cag gcc cca cta 1824
Pro Asp Pro Glu Leu Glu Ala Gly Ser Ala Pro Val Gln Ala Pro Leu
        595                 600                 605

gcc ttg gtc cct gag gag gcc gag agg aaa gaa gcc tcc agc tgc tcc 1872
Ala Leu Val Pro Glu Glu Ala Glu Arg Lys Glu Ala Ser Ser Cys Ser
    610                 615                 620

tac cag cag atc aac tgc ctg gac agc atc ctc agg tac ctg gag agc 1920
Tyr Gln Gln Ile Asn Cys Leu Asp Ser Ile Leu Arg Tyr Leu Glu Ser
625                 630                 635                 640

tgc aac ctc ccc agc acc act aag cgt aaa tgt gcc tcc tcc tcc tcc 1968
Cys Asn Leu Pro Ser Thr Thr Lys Arg Lys Cys Ala Ser Ser Ser Ser
                645                 650                 655

tat acc acc tcc tca gcc tct gac gac gac agg cag agg aca ggt cca 2016
Tyr Thr Thr Ser Ser Ala Ser Asp Asp Asp Arg Gln Arg Thr Gly Pro
            660                 665                 670

gtc tct gtg ggg acc aag aaa gat ccg ccg tca gca gcg ctg tct ggg 2064
Val Ser Val Gly Thr Lys Lys Asp Pro Pro Ser Ala Ala Leu Ser Gly
        675                 680                 685

gag ggg gcc acc cca cgg aag gag cca gtg gtg gga ggc acc ctg agc 2112
Glu Gly Ala Thr Pro Arg Lys Glu Pro Val Val Gly Gly Thr Leu Ser
    690                 695                 700

ccg ctc gcc ctg gcc aat aag gcg gag agt gtg gtg tcc gtc acc agt 2160
Pro Leu Ala Leu Ala Asn Lys Ala Glu Ser Val Val Ser Val Thr Ser
705                 710                 715                 720

cag tgt agc ttc agc tcc acc atc gtc cat gtg gga gac aag aag ccc 2208
Gln Cys Ser Phe Ser Ser Thr Ile Val His Val Gly Asp Lys Lys Pro
                725                 730                 735

ccg gag tcg gac atc atc atg atg gag gac ctg cct ggc cta gcc cca 2256
Pro Glu Ser Asp Ile Ile Met Met Glu Asp Leu Pro Gly Leu Ala Pro
            740                 745                 750

ggc cca gcc ccc agc cca gcc ccc agc ccc aca gta gcc cct gac cca 2304
Gly Pro Ala Pro Ser Pro Ala Pro Ser Pro Thr Val Ala Pro Asp Pro
        755                 760                 765

gcc cca gac gcc tac cgt cca gtg ggg ctg acc aag gcc gtg ctg tcc 2352
Ala Pro Asp Ala Tyr Arg Pro Val Gly Leu Thr Lys Ala Val Leu Ser
    770                 775                 780

ctg cac aca cag aag gaa gag caa gcc ttc ctc agc cgc ttc cga gac 2400
Leu His Thr Gln Lys Glu Glu Gln Ala Phe Leu Ser Arg Phe Arg Asp
785                 790                 795                 800

ctg ggc agg ctg cgt gga ctc gac agc tct tcc aca gct ccc tca gcc 2448

Leu Gly Arg Leu Arg Gly Leu Asp Ser Ser Ser Thr Ala Pro Ser Ala
                805                 810                 815

ctt ggc gag cga ggc tgc cac cac ggc ccc gca ccc cca agc cgc cga 2496
Leu Gly Glu Arg Gly Cys His His Gly Pro Ala Pro Pro Ser Arg Arg
            820                 825                 830

cac cac tgc cga tcc aaa gcc aag cgc tca cgc cac cac cag aac cct 2544
His His Cys Arg Ser Lys Ala Lys Arg Ser Arg His His Gln Asn Pro
        835                 840                 845

cgg gct gaa gcg ccc tgc tat gtc tca cac ccc tca ccc gtg cca ccc 2592
Arg Ala Glu Ala Pro Cys Tyr Val Ser His Pro Ser Pro Val Pro Pro
    850                 855                 860

tcc acc ccc tgg ccc acc cca cca gcc act acc ccc ttc cca gcg gtt 2640
Ser Thr Pro Trp Pro Thr Pro Pro Ala Thr Thr Pro Phe Pro Ala Val
865                 870                 875                 880

gtc cag ccc tac cct ctc cca gtg ttc tct cct cga gga ggc ccc cag 2688
Val Gln Pro Tyr Pro Leu Pro Val Phe Ser Pro Arg Gly Gly Pro Gln
                885                 890                 895

cct ctt ccc cct gct ccc aca tct gtg ccc cca gct gct ttc ccc gcc 2736
Pro Leu Pro Pro Ala Pro Thr Ser Val Pro Pro Ala Ala Phe Pro Ala
            900                 905                 910

cct ttg gtg acc cca atg gtg gcc ttg gtg ctc cct aac tat ctg ttc 2784
Pro Leu Val Thr Pro Met Val Ala Leu Val Leu Pro Asn Tyr Leu Phe
        915                 920                 925

cca acc cca tcc agc tat cct tat ggg gca ctc cag acc cct gct gaa 2832
Pro Thr Pro Ser Ser Tyr Pro Tyr Gly Ala Leu Gln Thr Pro Ala Glu
    930                 935                 940

ggg cct ccc act cct gcc tcg cac tcc cct tct cca tcc ttg ccc gcc 2880
Gly Pro Pro Thr Pro Ala Ser His Ser Pro Ser Pro Ser Leu Pro Ala
945                 950                 955                 960

ctc ccc ccg agt cct cct cac cgc ccg gac tct cca ctg ttc aac tcg 2928
Leu Pro Pro Ser Pro Pro His Arg Pro Asp Ser Pro Leu Phe Asn Ser
                965                 970                 975

aga tgc agc tct cca ctc cag ctc aat ctg ctg cag ctg gag gag ctc 2976
Arg Cys Ser Ser Pro Leu Gln Leu Asn Leu Leu Gln Leu Glu Glu Leu
            980                 985                 990

ccc cgt gct gag ggg gct gct gtt gca gga ggc cct ggg agc agt gcc 3024
Pro Arg Ala Glu Gly Ala Ala Val Ala Gly Gly Pro Gly Ser Ser Ala
        995                 1000                1005

ggg ccc cca cct ccc agt gcg gag gct gct gag cca gag gcc aga ctg 3072
Gly Pro Pro Pro Pro Ser Ala Glu Ala Ala Glu Pro Glu Ala Arg Leu
    1010                1015                1020

gcg gag gtc act gag tcc tcc aat cag gac gca ctt tcc ggc tcc agt 3120
Ala Glu Val Thr Glu Ser Ser Asn Gln Asp Ala Leu Ser Gly Ser Ser
1025                1030                1035                1040

gac ctg ctc gaa ctt ctg ctg caa gag gac tcg cgc tcc ggc aca ggc 3168
Asp Leu Leu Glu Leu Leu Leu Gln Glu Asp Ser Arg Ser Gly Thr Gly
                1045                1050                1055

tcc gca gcc tcg ggc tcc ttg ggc tct ggc ttg ggc tct ggg tct ggt 3216
Ser Ala Ala Ser Gly Ser Leu Gly Ser Gly Leu Gly Ser Gly Ser Gly
            1060                1065                1070

tca ggc tcc cat gaa ggg ggc agc acc tca gcc agc atc act cgc agc 3264
Ser Gly Ser His Glu Gly Gly Ser Thr Ser Ala Ser Ile Thr Arg Ser
        1075                1080                1085

agc cag agc agc cac aca agc aaa tac ttt ggc agc atc gac tct tcc 3312
Ser Gln Ser Ser His Thr Ser Lys Tyr Phe Gly Ser Ile Asp Ser Ser
    1090                1095                1100

gag gct gag gct ggg gct gct cgg ggc ggg gct gag cct ggg gac cag 3360

Glu Ala Glu Ala Gly Ala Ala Arg Gly Gly Ala Glu Pro Gly Asp Gln
1105                1110                1115                1120

gtg att aag tac gtg ctc cag gat ccc att tgg ctg ctc atg gcc aat 3408
Val Ile Lys Tyr Val Leu Gln Asp Pro Ile Trp Leu Leu Met Ala Asn
                1125                1130                1135

gct gac cag cgc gtc atg atg acc tac cag gtg ccc tcc agg gac atg 3456
Ala Asp Gln Arg Val Met Met Thr Tyr Gln Val Pro Ser Arg Asp Met
            1140                1145                1150

acc tct gtg ctg aag cag gat cgg gag cgg ctc cga gcc atg cag aag 3504
Thr Ser Val Leu Lys Gln Asp Arg Glu Arg Leu Arg Ala Met Gln Lys
        1155                1160                1165

cag cag cct cgg ttt tct gag gac cag cgg cgg gaa ctg ggt gct gtg 3552
Gln Gln Pro Arg Phe Ser Glu Asp Gln Arg Arg Glu Leu Gly Ala Val
    1170                1175                1180

cac tcc tgg gtc cgg aag ggc caa ctg cct cgg gct ctt gat gtg atg 3600
His Ser Trp Val Arg Lys Gly Gln Leu Pro Arg Ala Leu Asp Val Met
1185                1190                1195                1200

gcc tgt gtg gac tgt ggg agc agc acc caa gat cct ggt cac cct gat 3648
Ala Cys Val Asp Cys Gly Ser Ser Thr Gln Asp Pro Gly His Pro Asp
                1205                1210                1215

gac cca ctc ttc tca gag ctg gat gga ctg ggg ctg gag ccc atg gaa 3696
Asp Pro Leu Phe Ser Glu Leu Asp Gly Leu Gly Leu Glu Pro Met Glu
            1220                1225                1230

gag ggt gga ggc gag cag ggc agc agc ggt ggc ggc agt ggt gag gga 3744
Glu Gly Gly Gly Glu Gln Gly Ser Ser Gly Gly Gly Ser Gly Glu Gly
        1235                1240                1245

gag ggc tgc gag gag gcc caa ggc ggg gcc aag gct tca agc tct cag 3792
Glu Gly Cys Glu Glu Ala Gln Gly Gly Ala Lys Ala Ser Ser Ser Gln
    1250                1255                1260

gac ttg gct atg gag gag gag gaa gaa ggc agg agc tca tcc agt cca 3840
Asp Leu Ala Met Glu Glu Glu Glu Glu Gly Arg Ser Ser Ser Ser Pro
1265                1270                1275                1280

gcc tta cct aca gca gga aac tgc acc agc tag 3873
Ala Leu Pro Thr Ala Gly Asn Cys Thr Ser
                1285                1290

<210> 4
<211> 3876
<212> DNA
<213> Mus musculus

<220>
<221> CDS
<222> (1)..(3876)

<400> 4
atg agt ggt ccc cta gaa ggg gcc gat ggg gga gga gac ccc agg ccc 48
Met Ser Gly Pro Leu Glu Gly Ala Asp Gly Gly Gly Asp Pro Arg Pro
1               5                   10                  15

gga gaa cct ttt tgt cct gga gga gtc cca tcc cct ggg gcc ccg cag 96
Gly Glu Pro Phe Cys Pro Gly Gly Val Pro Ser Pro Gly Ala Pro Gln
            20                  25                  30

cac cgg cct tgt cca ggc ccc agc ctg gct gat gac act gat gca aac 144
His Arg Pro Cys Pro Gly Pro Ser Leu Ala Asp Asp Thr Asp Ala Asn
        35                  40                  45

agc aat ggc tca agt ggc aat gag tcc aac gga ccc gag tcc agg ggc 192
Ser Asn Gly Ser Ser Gly Asn Glu Ser Asn Gly Pro Glu Ser Arg Gly
    50                  55                  60

gca tct cag cgg agt tct cat agt tcc tct tct ggc aat ggc aag gac 240
Ala Ser Gln Arg Ser Ser His Ser Ser Ser Ser Gly Asn Gly Lys Asp
65                  70                  75                  80

tca gct ctg ctg gag acc act gag agc agc aag agt aca aac tca cag 288
Ser Ala Leu Leu Glu Thr Thr Glu Ser Ser Lys Ser Thr Asn Ser Gln
                85                  90                  95

agc cca tcc cca ccc agc agc tcc att gcc tac agc ctc ctg agt gcg 336
Ser Pro Ser Pro Pro Ser Ser Ser Ile Ala Tyr Ser Leu Leu Ser Ala
            100                 105                 110

agc tca gag cag gac aac cca tct acc agt ggc tgc agc agt gaa cag 384
Ser Ser Glu Gln Asp Asn Pro Ser Thr Ser Gly Cys Ser Ser Glu Gln
        115                 120                 125

tca gct cga gcc agg acc cag aaa gaa ctc atg act gca ctt cgg gag 432
Ser Ala Arg Ala Arg Thr Gln Lys Glu Leu Met Thr Ala Leu Arg Glu
    130                 135                 140

ctc aaa ctt cga ctg cca cca gag cgt cgg ggc aag ggc cgc tct ggg 480
Leu Lys Leu Arg Leu Pro Pro Glu Arg Arg Gly Lys Gly Arg Ser Gly
145                 150                 155                 160

acc ttg gcc aca ctg cag tac gct ctg gcc tgt gtc aag cag gtt cag 528
Thr Leu Ala Thr Leu Gln Tyr Ala Leu Ala Cys Val Lys Gln Val Gln
                165                 170                 175

gct aac cag gaa tat tac cag cag tgg agt ctg gag gag ggt gag cct 576
Ala Asn Gln Glu Tyr Tyr Gln Gln Trp Ser Leu Glu Glu Gly Glu Pro
            180                 185                 190

tgt gcc atg gac atg tct act tac acc ctg gag gaa ttg gag cat atc 624
Cys Ala Met Asp Met Ser Thr Tyr Thr Leu Glu Glu Leu Glu His Ile
        195                 200                 205

aca tcc gaa tac aca ctt cga aac cag gac acc ttc tct gtg gct gtg 672
Thr Ser Glu Tyr Thr Leu Arg Asn Gln Asp Thr Phe Ser Val Ala Val
    210                 215                 220

tcc ttc ctg aca ggc cgg att gtc tat att tcg gag cag gca ggt gtc 720

Ser Phe Leu Thr Gly Arg Ile Val Tyr Ile Ser Glu Gln Ala Gly Val
225                 230                 235                 240

ctg ctg cgt tgc aaa cgg gat gtg ttt cgg ggt gcc cgc ttc tca gag      768
Leu Leu Arg Cys Lys Arg Asp Val Phe Arg Gly Ala Arg Phe Ser Glu
                245                 250                 255

ctc ctg gct ccc cag gat gtg ggt gtc ttc tat ggc tct act aca cca      816
Leu Leu Ala Pro Gln Asp Val Gly Val Phe Tyr Gly Ser Thr Thr Pro
            260                 265                 270

tct cga ctg ccc acc tgg ggc act ggc acc tct gca ggt tca ggt ctc      864
Ser Arg Leu Pro Thr Trp Gly Thr Gly Thr Ser Ala Gly Ser Gly Leu
        275                 280                 285

aag gac ttc acc cag gaa aag tct gtc ttc tgc cga atc aga gga ggt      912
Lys Asp Phe Thr Gln Glu Lys Ser Val Phe Cys Arg Ile Arg Gly Gly
    290                 295                 300

cct gac cgg gat cca ggg cct cgg tac cag cca ttc cgc cta acc cca      960
Pro Asp Arg Asp Pro Gly Pro Arg Tyr Gln Pro Phe Arg Leu Thr Pro
305                 310                 315                 320

tat gtg acc aag att cgg gtc tca gat gga gcc cct gca cag ccg tgc      1008
Tyr Val Thr Lys Ile Arg Val Ser Asp Gly Ala Pro Ala Gln Pro Cys
                325                 330                 335

tgc cta ctc att gcc gag cgc atc cac tct ggt tat gaa gct ccc cgg      1056
Cys Leu Leu Ile Ala Glu Arg Ile His Ser Gly Tyr Glu Ala Pro Arg
            340                 345                 350

atc cct cct gac aag agg atc ttc acc acc cga cac aca cca agc tgc      1104
Ile Pro Pro Asp Lys Arg Ile Phe Thr Thr Arg His Thr Pro Ser Cys
        355                 360                 365

ctc ttc cag gat gta gat gaa agg gct gcc cca ctg ctg ggt tac ctt      1152
Leu Phe Gln Asp Val Asp Glu Arg Ala Ala Pro Leu Leu Gly Tyr Leu
    370                 375                 380

ccc cag gat ctc ctg ggg gct cca gta ctt ctc ttt cta cat cct gag 1200
Pro Gln Asp Leu Leu Gly Ala Pro Val Leu Leu Phe Leu His Pro Glu
385                 390                 395                 400

gac cga ccc ctc atg ctg gcc att cat aag aag ata ctg cag ctg gca 1248
Asp Arg Pro Leu Met Leu Ala Ile His Lys Lys Ile Leu Gln Leu Ala
                405                 410                 415

ggc cag ccc ttt gac cat tcc cct att cgc ttc tgt gct cgg aac ggg 1296
Gly Gln Pro Phe Asp His Ser Pro Ile Arg Phe Cys Ala Arg Asn Gly
            420                 425                 430

gaa tat gtc acc atg gac acc agc tgg gcc ggt ttt gtg cac ccc tgg 1344
Glu Tyr Val Thr Met Asp Thr Ser Trp Ala Gly Phe Val His Pro Trp
        435                 440                 445

agc cgc aag gtg gct ttc gtg ttg ggt cgc cat aaa gtg cgc acg gca 1392
Ser Arg Lys Val Ala Phe Val Leu Gly Arg His Lys Val Arg Thr Ala
    450                 455                 460

ccc ctg aat gag gac gtc ttc act ccc cca gcc ccc agc cca gct ccg 1440
Pro Leu Asn Glu Asp Val Phe Thr Pro Pro Ala Pro Ser Pro Ala Pro
465                 470                 475                 480

tcc ctg gac tct gat atc cag gag ctc tca gag cag atc cat cga ttg 1488
Ser Leu Asp Ser Asp Ile Gln Glu Leu Ser Glu Gln Ile His Arg Leu
                485                 490                 495

ctg ctg cag cct gtg cac agc tcc agc ccc acg ggg ctc tgt gga gtt 1536
Leu Leu Gln Pro Val His Ser Ser Ser Pro Thr Gly Leu Cys Gly Val
            500                 505                 510

ggc cct ctg atg tcc cct ggt cct cta cac agc cct ggc tcc tcc agt 1584
Gly Pro Leu Met Ser Pro Gly Pro Leu His Ser Pro Gly Ser Ser Ser
        515                 520                 525

gat agc aat ggg ggg gac gct gag ggg cct ggg cct cct gct cca gtg 1632

Asp Ser Asn Gly Gly Asp Ala Glu Gly Pro Gly Pro Pro Ala Pro Val
    530                 535                 540

act ttc cag cag atc tgt aag gat gtg cat ctg gta aag cac cag gga 1680
Thr Phe Gln Gln Ile Cys Lys Asp Val His Leu Val Lys His Gln Gly
545                 550                 555                 560

caa cag ctc ttc att gaa tct cgg gcc aag ccc cca ccc cgg ccc cgc 1728
Gln Gln Leu Phe Ile Glu Ser Arg Ala Lys Pro Pro Pro Arg Pro Arg
                565                 570                 575

ctc ctt gct aca ggt aca ttc aaa gcc aaa gtc ctt ccc tgc cag tcc 1776
Leu Leu Ala Thr Gly Thr Phe Lys Ala Lys Val Leu Pro Cys Gln Ser
            580                 585                 590

cca aac ccc gaa ctg gag gtg gcc cca gtt cct gac caa gcc tcg tta 1824
Pro Asn Pro Glu Leu Glu Val Ala Pro Val Pro Asp Gln Ala Ser Leu
        595                 600                 605

gcc ttg gcc cct gag gag cca gag agg aaa gaa acc tct ggc tgt tcc 1872
Ala Leu Ala Pro Glu Glu Pro Glu Arg Lys Glu Thr Ser Gly Cys Ser
    610                 615                 620

tac cag cag atc aac tgc ctg gac agc atc ctc agg tat ttg gag agc 1920
Tyr Gln Gln Ile Asn Cys Leu Asp Ser Ile Leu Arg Tyr Leu Glu Ser
625                 630                 635                 640

tgc aac att ccc agt aca acc aag cgt aaa tgt gcc tcc tcc tcc tcc 1968
Cys Asn Ile Pro Ser Thr Thr Lys Arg Lys Cys Ala Ser Ser Ser Ser
                645                 650                 655

tac act gcc tct tca gcc tct gat gat gac aag cag agg gca ggt cca 2016
Tyr Thr Ala Ser Ser Ala Ser Asp Asp Asp Lys Gln Arg Ala Gly Pro
            660                 665                 670

gtt cct gtg ggg gcc aag aaa gat ccg tcg tca gca atg ctg tct ggg 2064
Val Pro Val Gly Ala Lys Lys Asp Pro Ser Ser Ala Met Leu Ser Gly
        675                 680                 685

gag ggg gca act cct cgg aag gag cca gtg gtg gga ggc acc ctg agc 2112
Glu Gly Ala Thr Pro Arg Lys Glu Pro Val Val Gly Gly Thr Leu Ser
    690                 695                 700

ccg ctc gcc ctg gcc aat aag gca gag agc gtg gtg tcc gtc acc agt 2160
Pro Leu Ala Leu Ala Asn Lys Ala Glu Ser Val Val Ser Val Thr Ser
705                 710                 715                 720

cag tgt agc ttc agc tcc acc atc gtc cat gtg gga gac aag aag ccc 2208
Gln Cys Ser Phe Ser Ser Thr Ile Val His Val Gly Asp Lys Lys Pro
                725                 730                 735

ccg gag tcg gac atc atc atg atg gaa gac ctg cct ggc ctg gcc cct 2256
Pro Glu Ser Asp Ile Ile Met Met Glu Asp Leu Pro Gly Leu Ala Pro
            740                 745                 750

ggc cca gcc ccc agt ccg gcc ccc agc ccc aca gta gcc cct gac cca 2304
Gly Pro Ala Pro Ser Pro Ala Pro Ser Pro Thr Val Ala Pro Asp Pro
        755                 760                 765

acc cca gat gct tat cgc cca gtg ggt ctg acc aag gcc gtg ctg tcc 2352
Thr Pro Asp Ala Tyr Arg Pro Val Gly Leu Thr Lys Ala Val Leu Ser
    770                 775                 780

ctg cac aca cag aag gaa gag caa gcc ttc ctc aac cgc ttc aga gat 2400
Leu His Thr Gln Lys Glu Glu Gln Ala Phe Leu Asn Arg Phe Arg Asp
785                 790                 795                 800

ctt ggc agg ctt cgt gga ctt gac acc tct tct gtg gcc ccc tca gcc 2448
Leu Gly Arg Leu Arg Gly Leu Asp Thr Ser Ser Val Ala Pro Ser Ala
                805                 810                 815

cct ggc tgc cac cat ggc ccc att ccc cct ggt cgc cga cac cac tgc 2496
Pro Gly Cys His His Gly Pro Ile Pro Pro Gly Arg Arg His His Cys
            820                 825                 830

cga tct aaa gca aag cgt tcc cgc cac cac cac cac cag acc ccc cgg 2544

Arg Ser Lys Ala Lys Arg Ser Arg His His His His Gln Thr Pro Arg
        835                 840                 845

ccc gaa act ccc tgc tat gtc tcc cat cct tca cct gtg ccc tct tct      2592
Pro Glu Thr Pro Cys Tyr Val Ser His Pro Ser Pro Val Pro Ser Ser
    850                 855                 860

gga ccc tgg cca ccc cca cca gcc acg acc ccc ttc cca gca atg gtc      2640
Gly Pro Trp Pro Pro Pro Pro Ala Thr Thr Pro Phe Pro Ala Met Val
865                 870                 875                 880

cag ccc tac cca ctc cca gta ttc tcc cct cga gga gga ccc cag ccc      2688
Gln Pro Tyr Pro Leu Pro Val Phe Ser Pro Arg Gly Gly Pro Gln Pro
                885                 890                 895

ctt ccc cct gcc cct aca tct gtg tcc cct gct acc ttc cct tct ccc      2736
Leu Pro Pro Ala Pro Thr Ser Val Ser Pro Ala Thr Phe Pro Ser Pro
            900                 905                 910

tta gtg acc cca atg gtg gcc ttg gtg ctc cct aac tat cta ttc cct      2784
Leu Val Thr Pro Met Val Ala Leu Val Leu Pro Asn Tyr Leu Phe Pro
        915                 920                 925

acc cca cct agt tat cca tat ggg gtg tcc cag gcc cct gtt gag ggg      2832
Thr Pro Pro Ser Tyr Pro Tyr Gly Val Ser Gln Ala Pro Val Glu Gly
    930                 935                 940

cca ccc acg cct gct tcc cac tcg ccc tct cca tcc ctg ccc cca cca      2880
Pro Pro Thr Pro Ala Ser His Ser Pro Ser Pro Ser Leu Pro Pro Pro
945                 950                 955                 960

cct ctc agc ccc ccc cac cgc cca gac tcc cca ctg ttc aac tcg aga      2928
Pro Leu Ser Pro Pro His Arg Pro Asp Ser Pro Leu Phe Asn Ser Arg
                965                 970                 975

tgc agc tcc cca ctc cag ctc aat ctg ctg cag ctt gag gag tcc ccc      2976
Cys Ser Ser Pro Leu Gln Leu Asn Leu Leu Gln Leu Glu Glu Ser Pro
            980                 985                 990

cgc acg gag ggg ggc gct gct gca gga ggc cca gga agc agt gct ggg     3024
Arg Thr Glu Gly Gly Ala Ala Ala Gly Gly Pro Gly Ser Ser Ala Gly
        995                 1000                1005

ccc ctg cct ccc agt gag gag act gct gag cca gag gcc aga ttg gtg     3072
Pro Leu Pro Pro Ser Glu Glu Thr Ala Glu Pro Glu Ala Arg Leu Val
    1010                1015                1020

gag gtt act gag tcg tcc aat cag gat gca ctt tca ggc tcc agc gac     3120
Glu Val Thr Glu Ser Ser Asn Gln Asp Ala Leu Ser Gly Ser Ser Asp
1025                1030                1035                1040

ctg ctg gag cta ctg ctc caa gaa gac tct cgc tcg ggc aca ggc tcc     3168
Leu Leu Glu Leu Leu Leu Gln Glu Asp Ser Arg Ser Gly Thr Gly Ser
                1045                1050                1055

gca gcc tca ggc tcc ctg ggc tct ggc ctg ggc tct ggg tct ggt tca     3216
Ala Ala Ser Gly Ser Leu Gly Ser Gly Leu Gly Ser Gly Ser Gly Ser
            1060                1065                1070

gga tcc cac gaa ggg gga agc acc tca gcc agc atc acc cgc agc agt     3264
Gly Ser His Glu Gly Gly Ser Thr Ser Ala Ser Ile Thr Arg Ser Ser
        1075                1080                1085

cag agc agc cat aca agc aag tac ttt ggc agc atc gac tct tcc gag     3312
Gln Ser Ser His Thr Ser Lys Tyr Phe Gly Ser Ile Asp Ser Ser Glu
    1090                1095                1100

gct gaa gct ggg gct gct cgg gcc agg act gag cct ggg gac cag gtc     3360
Ala Glu Ala Gly Ala Ala Arg Ala Arg Thr Glu Pro Gly Asp Gln Val
1105                1110                1115                1120

att aag tgt gtg ctc cag gac ccc atc tgg ctg ctc atg gcc aat gcc     3408
Ile Lys Cys Val Leu Gln Asp Pro Ile Trp Leu Leu Met Ala Asn Ala
                1125                1130                1135

gac cag cgt gtc atg atg aca tac cag gtg ccg tcc agg gat gca gcc     3456

Asp Gln Arg Val Met Met Thr Tyr Gln Val Pro Ser Arg Asp Ala Ala
            1140                1145                1150

tct gtg ctg aag caa gac cgg gag agg ctc cgg gcc atg cag aaa cag      3504
Ser Val Leu Lys Gln Asp Arg Glu Arg Leu Arg Ala Met Gln Lys Gln
        1155                1160                1165

cag cca cgg ttc tca gag gac cag agg cgg gaa ctg ggt gct gtg cac      3552
Gln Pro Arg Phe Ser Glu Asp Gln Arg Arg Glu Leu Gly Ala Val His
    1170                1175                1180

tcc tgg gtc cgg aag ggc cag ctg cct cgg gcc ctt gat gtg atg gcg      3600
Ser Trp Val Arg Lys Gly Gln Leu Pro Arg Ala Leu Asp Val Met Ala
1185                1190                1195                1200

tgt gtg gac tgt ggc agc agc gtt caa gat cct ggc cac tct gat gac      3648
Cys Val Asp Cys Gly Ser Ser Val Gln Asp Pro Gly His Ser Asp Asp
                1205                1210                1215

ccg ctc ttc tca gaa ctg gat gga ttg ggg ctg gag ccc atg gaa gag      3696
Pro Leu Phe Ser Glu Leu Asp Gly Leu Gly Leu Glu Pro Met Glu Glu
            1220                1225                1230

ggt gga ggc gag ggt ggt ggg tgt ggt gtt ggc ggc ggt ggg ggt gat      3744
Gly Gly Gly Glu Gly Gly Gly Cys Gly Val Gly Gly Gly Gly Gly Asp
        1235                1240                1245

ggt ggt gag gag gcc cag acc caa att ggg gct aag ggt tca agc tct      3792
Gly Gly Glu Glu Ala Gln Thr Gln Ile Gly Ala Lys Gly Ser Ser Ser
    1250                1255                1260

cag gac tct gcc atg gag gaa gaa gag caa ggt ggg ggc tca tcc agc      3840
Gln Asp Ser Ala Met Glu Glu Glu Glu Gln Gly Gly Gly Ser Ser Ser
1265                1270                1275                1280

cca gct tta cct gca gaa gaa aac agc acc agc tag                      3876
Pro Ala Leu Pro Ala Glu Glu Asn Ser Thr Ser
                1285                1290